# EUROPEAN PATENT APPLICATION

(11) **EP 4 403 636 A1**
(43) Date of publication of application: **24.07.2024**
(21) Application number: 22867519.5
(22) Date of filing: 28.06.2022
(51) Int. Cl.: C12N 15/75, C12P 13/14, C12P 7/18, C12P 19/04, C12N 9/12, C12N 9/04, C12N 9/06, C12N 9/24, C12N 9/00

(54) **RECOMBINANT MICROORGANISM WITH CONTROLLED ABILITY TO PRODUCE POLYOLS OR EXOPOLYMERS**

(30) Priority: 13.09.2021 KR 20210121930
(71) Applicant: GS Caltex Corporation, Seoul 06141 (KR)
(72) Inventor: SONG, Chan Woo, Seoul 06141 (KR); KWON, Min-A, Seoul 06141 (KR); SONG, Hyo Hak, Seoul 06141 (KR); JEONG, Ki Woong, Seoul 06141 (KR); KIM, Seong Hyeon, Seoul 06141 (KR)
(74) Representative: Zacco Sweden AB
(86) International application number: PCT/KR2022/009268
(87) International publication number: WO 2023/038250

(57) **Abstract**

The present invention relates to a recombinant microorganism with the controlled ability to produce polyols or exopolymers. The present invention also relates to a selective production or simultaneous production method for polyols or exopolymers using the recombinant microorganism of the present invention.

## Description

### [Technical Field]

The present invention relates to a recombinant microorganism with controlled ability to produce polyols or exopolymers. Further, the present invention relates to a production method of polyols or exopolymers using the recombinant microorganism.

### [Background Art]

Bacillus licheniformis microorganism is classified as a generally regarded as safe (GRAS) microorganism, and is a microorganism that can produce various viscous polymers, for example, as industrial enzymes such as amylase and protease, polyglutamic acid, levan, and exopolysaccharide, and various polyols, for example, 2,3-Butanediol (2,3-BDO) and glycerol. Polyglutamic acid, levan, and exopolysaccharide, which are classified as viscous polymers, are high value-added materials that can be widely used as moisturizers in cosmetics, agricultural products, and foods. The polyols that Bacillus licheniformis can produce are largely 2,3-butanediol and glycerol. 2,3-butanediol, one (CH₃CHOHCHOHCH₃) of the alcohols with four carbons and two hydroxy groups (-OH), has three isomers consisting of (2R,3S)-butanediol, (2R,3S)-butanediol, and (2S, 3S)-butanediol, and it is a high value-added material that can be widely used as a moisturizer, plant disease inhibitor, and immunity enhancer in cosmetics, agricultural products, and food. In addition, glycerol is a polyol with three carbons and three hydroxy groups (-OH) and is widely used as a raw material for moisturizers in cosmetics and other products.

Research to produce viscous polymers such as polyglutamic acid, levan, and exopolysaccharide using Bacillus microorganisms has been conducted for a long time (Birrer et al., Int. J. Biol. Macromol., 16:265-275, 1994; Gojgic et al., Int. J. Biol. Macromol., 121:142-151, 2019;Asgher et al., Int. J. Biol. Macromol., 151:984-992,2020). Research on 2,3-butanediol production by various microorganisms such as Klebsiella pneumoniae, K. oxytoca, Bacillus licheniformis, and B. subtils has been reported Research on the development of various recombinant microorganisms has been conducted to improve 2,3-butanediol production yield and selectivity. For example, research on the development of microorganisms has been actively conducted mainly through reduction of by-products, such as by exposing microorganisms to UV or inhibiting the main by-product production pathways (Song et al. J. Ind. Microbial. Biotechnol., 46:1583-1601, 2019).

### [Disclosure]

### [Technical Problem]

An object of the present invention is to provide recombinant microorganisms with controlled ability to produce polyols or exopolymers.

Another object of the present invention is to provide a method for producing polyol using the recombinant microorganism.

Another object of the present invention is to provide a method for producing exopolymers using the above recombinant microorganisms.

### [Technical Solution]

In order to achieve the above object, the present invention provides a recombinant microorganism with controlled ability to produce polyol or exopolymer in a microorganism with a biosynthetic pathway for polyol or exopolymer, characterized in that one or more selected from the group consisting of a pathway that converts glucose-6-phosphate to exopolysaccharide, a pathway that converts glycerophosphate to glycerol, a pathway that converts acetoin to (2R,3S)-butanediol, a pathway that converts acetoin to (2R,3R)-butanediol, and a pathway that converts glutamic acid to polyglutamic acid are inhibited.

In addition, the present invention provides a method for producing polyol comprising the steps of preparing the recombinant microorganism of the present invention; and culturing the recombinant microorganism in a medium containing a carbon source.

In addition, the present invention provides a method for producing exopolymer comprising the steps of preparing the recombinant microorganism of the present invention; and culturing the recombinant microorganism in a medium containing a carbon source.

### [Advantageous Effects]

The recombinant microorganism of the present invention can simultaneously produce polyols and exopolymers, and can selectively suppress, not produce, or increase the production of specific types of polyols and/or exopolymers.

### [Description of Drawings]

FIG. 1 shows a metabolic pathway of the majorly produced exopolymers and polyols and a corresponding enzyme reaction.
FIG. 2 shows a method for simultaneous recovery of exopolymer and polyol produced from fermentation broth.

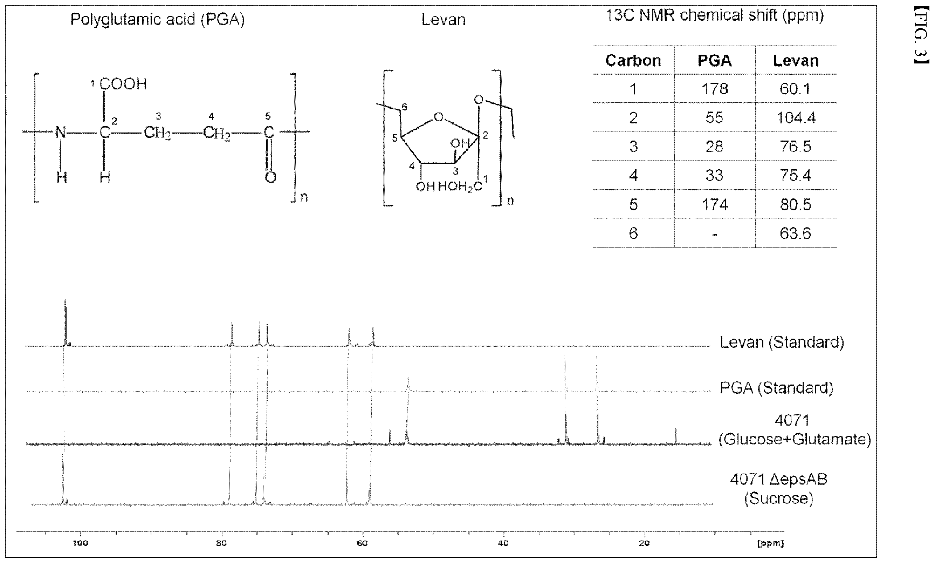

FIG. 3 shows a 13C-NMR based analysis method of levan and polyglutamic acid among exopolymers.

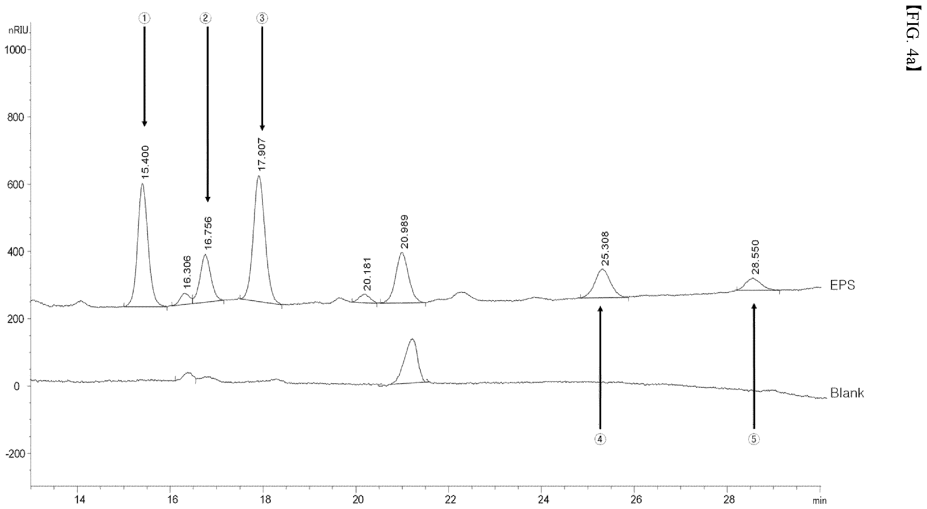

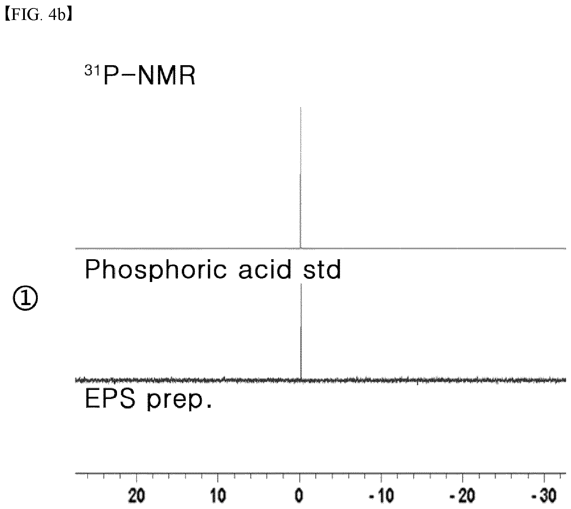

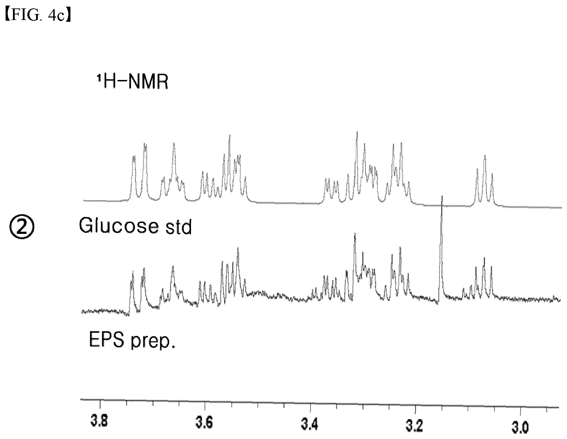

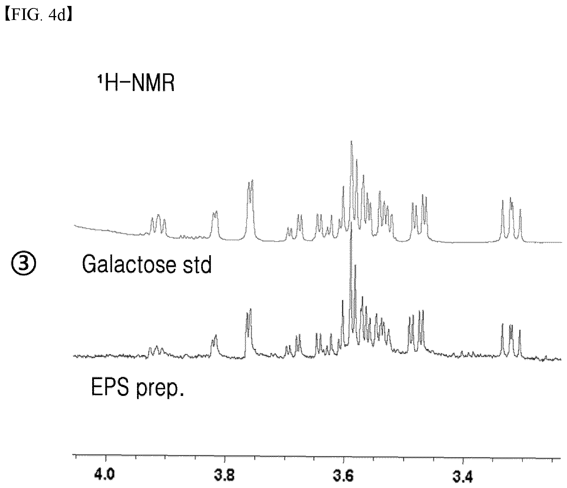

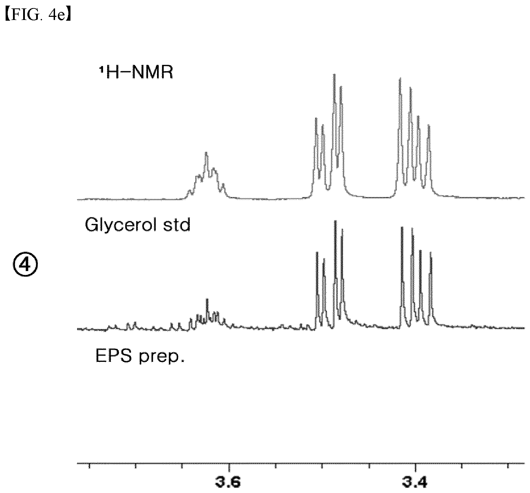

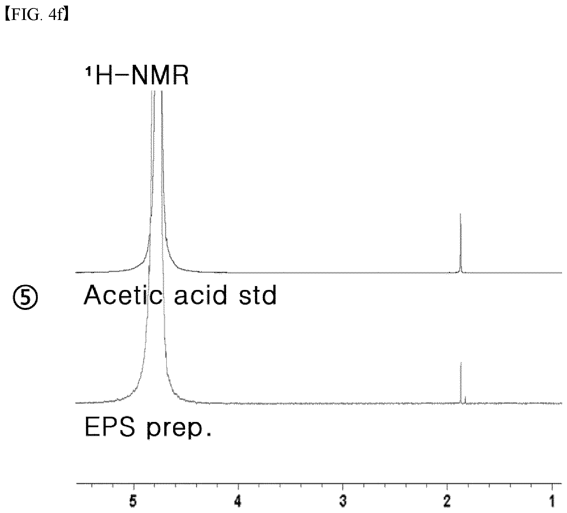

FIG. 4 shows a method for identifying monomers based on 13C-NMR, 1H-NMR, and 31P-NMR of (a) exopolysaccharides, (b) phosphoric acid, (c) glucose, (d) galactose, (e) glycerol, and (f) acetic acid, among viscous polymers.
FIG. 5 shows the amount and ratio of polyol produced by a wild-type microorganism of Comparative Example 1 and a recombinant microorganism of Example 1 depending on a carbon source in flask culture at low temperature conditions (30°C).
FIG. 6 shows the amount and ratio of exopolymer produced by a wild-type microorganism of Comparative Example 1 and a recombinant microorganism of Example 1 depending on a carbon source in flask culture at low temperature conditions (30°C).
FIG. 7 shows the amount and ratio of polyol produced by a wild-type microorganism of Comparative Example 1 and a recombinant microorganism of Example 1 depending on a carbon source in flask culture at low temperature conditions (45°C).
FIG. 8 shows changes in the amount of polyglutamic acid, exopolysaccharide, and polyol produced by a wild-type microorganism of Comparative Example 1 and a recombinant microorganism of Example 1 depending on a carbon source in flask culture at high temperature conditions (45°C).

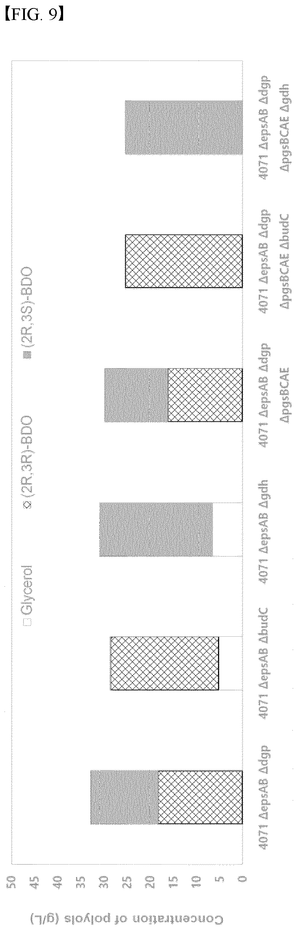

FIG. 9 shows the amount and ratio of polyol produced by a recombinant microorganism in flask culture at low temperature conditions (30°C) using sucrose as a carbon source.

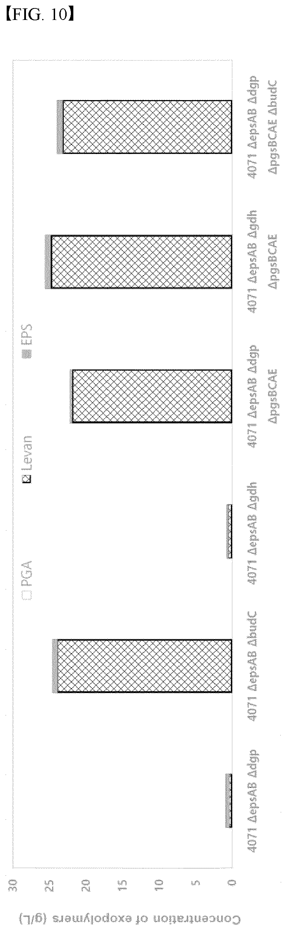

FIG. 10 shows the amount and ratio of levan produced by a recombinant microorganism in flask culture at low temperature conditions (30°C) using sucrose as a carbon source.

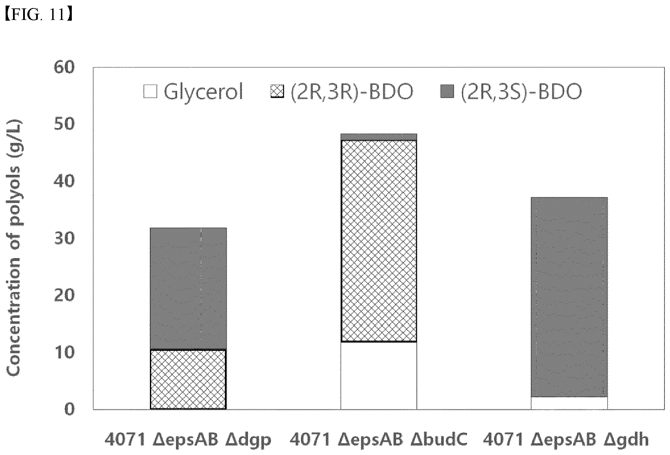

FIG. 11 shows the amount and ratio of polyol produced by a recombinant microorganism in flask culture at high temperature conditions (45°C) using glucose and glutamic acid as carbon sources.

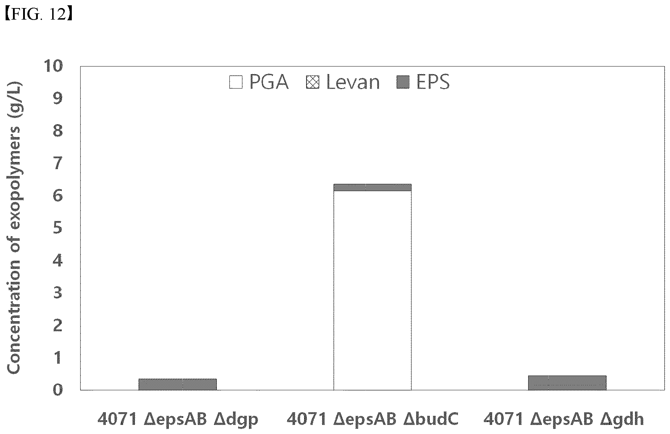

FIG. 12 shows the amount and ratio of polyglutamic acid produced by a recombinant microorganism in flask culture at high temperature conditions (45°C) using glucose and glutamic acid as carbon sources.

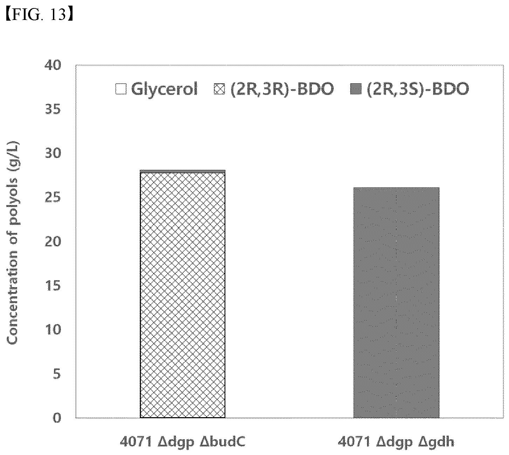

FIG. 13 shows the amount and ratio of polyol produced by a recombinant microorganisms in flask culture under low temperature conditions (30°C) using glucose as a carbon source.

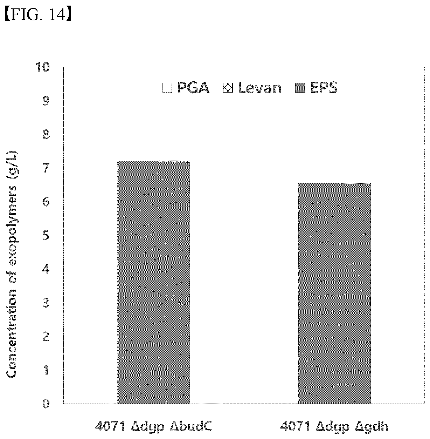

FIG. 14 shows the amount and ratio of EPS produced by a recombinant microorganism in flask culture under low temperature conditions (30°C) using glucose as a carbon source.
FIG. 15 shows the results of simultaneous production of (2R,3S)-butanediol and levan through fermentation of a recombinant microorganism of Example 8 under low-temperature culture conditions at 30°C using sucrose as a carbon source.
FIG. 16 shows the results of simultaneous production of (2R,3S)-butanediol and levan through fermentation of a recombinant microorganism of Example 8 under high temperature culture conditions at 45°C using sucrose as a carbon source.

### [Best Mode for Invention]

The present invention relates to a recombinant microorganism with controlled ability to produce polyol or exopolymer in a microorganism with a biosynthetic pathway for polyol or exopolymer, characterized in that one or more selected from the group consisting of a pathway that converts glucose-6-phosphate to exopolysaccharide, a pathway that converts glycerophosphate to glycerol, a pathway that converts acetoin to (2R,3S)-butanediol, a pathway that converts acetoin to (2R,3R)-butanediol, and a pathway that converts glutamic acid to polyglutamic acid are inhibited.

In addition, the present invention relates to a method for producing polyol comprising the steps of preparing the recombinant microorganism of the present invention; and culturing the recombinant microorganism in a medium containing a carbon source.

In addition, the present invention relates to a method for producing exopolymer comprising the steps of preparing the recombinant microorganism of the present invention; and culturing the recombinant microorganism in a medium containing a carbon source.

Hereinafter, the present invention will be described in detail.

### Recombinant microorganism with controlled ability to produce polyol or exopolymer

The present invention relates to a recombinant microorganism with controlled ability to produce polyol or exopolymer in a microorganism with a biosynthetic pathway for polyol or exopolymer, characterized in that one or more selected from the group consisting of a pathway that converts glucose-6-phosphate to exopolysaccharide, a pathway that converts glycerophosphate to glycerol, a pathway that converts acetoin to (2R,3S)-butanediol, a pathway that converts acetoin to (2R,3R)-butanediol, and a pathway that converts glutamic acid to polyglutamic acid are inhibited.

The recombinant microorganism may be a recombinant Bacillus, preferably a recombinant Bacillus licheniformis.

The recombinant microorganism of the present invention may have the ability to simultaneously produce polyol and exopolymer.

The polyol may be one or more selected from the group consisting of glycerol, (2R, 3R)-butanediol, and (2R, 3S)-butanediol.

The exopolymer may be one or more selected from the group consisting of polyglutamic acid, levan, and exopolysaccharide. The exopolysaccharide may include one or more monomers selected from the group consisting of glucose, galactose, phosphate, glycerol, and acetic acid.

The recombinant microorganism of the present invention may simultaneously produce polyol and exopolymer, and may selectively inhibit, not produce, or increase the production of specific types of polyol and/or exopolymer. Therefore, the recombinant microorganism of the present invention is capable of simultaneous and selective production of polyol and exopolymer.

Compared to wild-type microorganisms, the recombinant microorganism of the present invention may be a recombinant microorganism that has an ability to inhibit the production of one or more polyols selected from the group consisting of glycerol, (2R, 3R)-butanediol, and (2R,3S)-butanediol, or has no ability to produce the above one or more polyols.

In an embodiment, the recombinant microorganism of the present invention may be a recombinant microorganism in which a pathway that converts glucose-6-phosphate to exopolysaccharide is inhibited, and one or more selected from the group consisting of a pathway that converts glycerophosphate to glycerol, a pathway that converts acetoin to (2R,3S)-butanediol, a pathway that converts acetoin to (2R,3R)-butanediol, and a pathway that converts glutamic acid to polyglutamic acid are inhibited.

In an embodiment, the recombinant microorganism of the present invention may be a recombinant microorganism in which a pathway that converts glucose-6-phosphate to exopolysaccharide is inhibited, and may be a recombinant microorganism in which one or more selected from the group consisting of a pathway that converts glycerophosphate to glycerol, a pathway that converts acetoin to (2R,3S)-butanediol, a pathway that converts acetoin to (2R,3R)-butanediol, and a pathway that converts glutamic acid to polyglutamic acid are not inhibited. In this case, the recombinant microorganism may have a higher ability to produce total polyol, glycerol, (2R,3R)-butanediol, (2R,3S)-butanediol, total exopolymer, and/or levan than the wild-type microorganism. Also, in this case, the recombinant microorganism may have a lower ability to produce EPS than the wild-type microorganism.

In an embodiment, the recombinant microorganism of the present invention may be a recombinant microorganism in which a pathway that converts glucose-6-phosphate to exopolysaccharide and pathway that converts glycerophosphate to glycerol are inhibited, and may be a recombinant microorganism in which a pathway that converts acetoin to (2R,3S)-butanediol, a pathway that converts acetoin to (2R,3R)-butanediol, and a pathway that converts glutamic acid to polyglutamic acid are not inhibited. In this case, the recombinant microorganism may have a higher ability to produce (2R,3R)-butanediol and/or (2R,3S)-butanediol than the wild-type microorganism. Also, in this case, the recombinant microorganism may have a lower ability to produce total polyol, glycerol, total exopolymer, levan, and/or EPS than the wild-type microorganism.

In an embodiment, the recombinant microorganism of the present invention may be a recombinant microorganism in which a pathway that converts glucose-6-phosphate to exopolysaccharide and a pathway that converts acetoin to (2R,3S)-butanediol are inhibited, and may be a recombinant microorganism in which a pathway that converts glycerophosphate to glycerol, a pathway that converts acetoin to (2R,3R)-butanediol, and a pathway that converts glutamic acid to polyglutamic acid are not inhibited. In this case, the recombinant microorganism may have a higher ability to produce (2R,3R)-butanediol, total exopolymer, and/or levan than the wild-type microorganism. Also, in this case, the recombinant microorganism may have a lower ability to produce total polyol, glycerol, (2R,3 S)-butanediol and/or EPS than the wild-type microorganism.

In an embodiment, the recombinant microorganism of the present invention may be a recombinant microorganism in which a pathway that converts glucose-6-phosphate to exopolysaccharide and a pathway that converts acetoin to (2R,3R)-butanediol are inhibited, and may be a recombinant microorganism in which a pathway that converts glycerophosphate to glycerol, a pathway that converts acetoin to (2R,3S)-butanediol, and a pathway that converts glutamic acid to polyglutamic acid are not inhibited. In this case, the recombinant microorganism may have a higher ability to produce (2R,3S)-butanediol than the wild-type microorganism. Also, in this case, the recombinant microorganism may have a lower ability to produce total polyol, glycerol, (2R, 3R)-butanediol, total exopolymer, levan, and/or EPS than the wild-type microorganism.

In an embodiment, the recombinant microorganism of the present invention may be a recombinant microorganism in which a pathway that converts glucose-6-phosphate to exopolysaccharide, a pathway that converts glycerophosphate to glycerol, and a pathway that converts glutamic acid to polyglutamic acid are inhibited, and may be a recombinant microorganism in which a pathway that converts acetoin to (2R,3S)-butanediol and a pathway that converts acetoin to (2R,3R)-butanediol are not inhibited. In this case, the recombinant microorganism may have a higher ability to produce (2R,3R)-butanediol, total exopolymer, and/or levan than the wild-type microorganism. Also, in this case, the recombinant microorganism may have a lower ability to produce total polyol, glycerol, (2R,3S)-butanediol and/or EPS than the wild-type microorganism.

In an embodiment, the recombinant microorganism of the present invention may be a recombinant microorganism in which a pathway that converts glucose-6-phosphate to exopolysaccharide, a pathway that converts acetoin to (2R,3R)-butanediol, and a pathway that converts glutamic acid to polyglutamic acid are inhibited, and may be a recombinant microorganism in which a pathway that converts glycerophosphate to glycerol and a pathway that converts acetoin to (2R,3S)-butanediol are not inhibited. In this case, the recombinant microorganism may have a higher ability to produce (2R,3S)-butanediol, total exopolymer, and/or levan than the wild-type microorganism. Also, in this case, the recombinant microorganism may have a lower ability to produce total polyol, glycerol, (2R,3R)-butanediol and/or EPS than the wild-type microorganism.

In an embodiment, the recombinant microorganism of the present invention may be a recombinant microorganism in which a pathway that converts glucose-6-phosphate to exopolysaccharide, a pathway that converts glycerophosphate to glycerol, a pathway that converts glutamic acid to polyglutamic acid, and a pathway that converts acetoin to (2R,3 S)-butanediol are inhibited, and may be a recombinant microorganism in which a pathway that converts acetoin to (2R,3R)-butanediol is not inhibited. In this case, the recombinant microorganism may have a higher ability to produce (2R,3R)-butanediol, total exopolymer, and/or levan than the wild-type microorganism. Also, in this case, the recombinant microorganism may have a lower ability to produce total polyol, glycerol, (2R,3 S)-butanediol and/or EPS than the wild-type microorganism.

In an embodiment, the recombinant microorganism of the present invention may be a recombinant microorganism in which a pathway that converts glucose-6-phosphate to exopolysaccharide, a pathway that converts glycerophosphate to glycerol, a pathway that converts glutamic acid to polyglutamic acid, and a pathway that converts acetoin to (2R,3R)-butanediol are inhibited, and may be a recombinant microorganism in which a pathway that converts acetoin to (2R,3S)-butanediol is not inhibited. In this case, the recombinant microorganism may have a higher ability to produce (2R,3S)-butanediol, total exopolymer, and/or levan than the wild-type microorganism. Also, in this case, the recombinant microorganism may have a lower ability to produce total polyol, glycerol, (2R,3R)-butanediol, and/or EPS than the wild-type microorganism.

### Polyol biosynthesis pathway

The polyol biosynthetic pathway of the present invention refers to a pathway in which polyol is synthesized from specific metabolites within microorganisms. For example, the pathway where glycerol is synthesized from specific metabolites within microorganisms, the pathway where (2R,3R)-butanediol is synthesized from specific metabolites within microorganisms, the pathway where (2R,3S)-butanediol is synthesized from specific metabolites within microorganisms, etc.

### Biosynthetic pathway of exopolymer

The biosynthetic pathway of the exopolymer of the present invention refers to a pathway in which exopolymer is synthesized from specific metabolites within microorganisms. For example, it may be a pathway where polyglutamic acid is synthesized from a specific metabolite within a microorganism, a pathway where levan is synthesized from a specific metabolite within a microorganism, a pathway where exopolysaccharide is synthesized from a specific metabolite within a microorganism, etc.

### Microorganisms with a biosynthetic pathway for polyol or exopolymer

Microorganisms having a biosynthetic pathway for polyol or exopolymer of the present invention are microorganisms having the biosynthetic pathways described above, and are preferably microorganisms having a biosynthetic pathway for polyols and exopolymer. The microorganism may be a microorganism that has a wild-type biosynthetic pathway for polyol or exopolymer, or a recombinant microorganism that has the biosynthetic pathway through genetic recombination. For example, the microorganism of the present invention may be a microorganism of the genus Klebsiella, Bacillus, Serratia, or Enterobacter, and is preferably Bacillus licheniformis (B. licheniformis), Bacillus subtilis (B. subtilis), Bacillus amyloliquefaciens (B. amyloliquefaciens), Pennybacillus polymyxa (P. polymyxa), etc., is most preferably Bacillus licheniformis (B. licheniformis). Preparing the recombinant microorganism of the present invention using Bacillus licheniformis is advantageous for industrial-scale production of exopolymer and/or polyol.

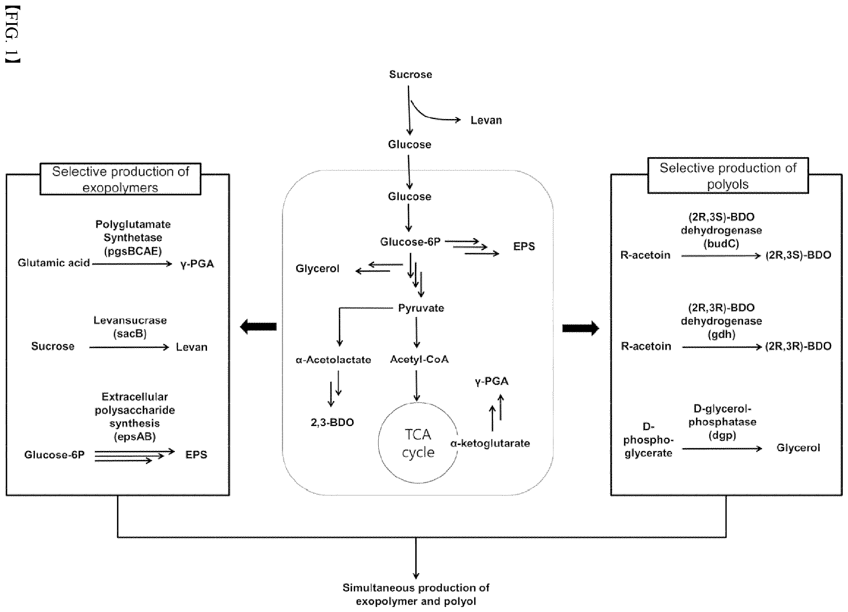

FIG. 1 shows the metabolic pathway of exopolymer and polyol mainly produced in the microorganism of the present invention, preferably Bacillus licheniformis, and the corresponding enzymatic reactions.

### Inhibition of a pathway that converts glucose-6-phosphate to exopolysaccharide

An exopolysaccharide synthesis enzyme regulates the conversion of glucose-6-phosphate to EPS. The pathway that converts glucose-6-phosphate to exopolysaccharide may be inhibited by deleting the epsAB gene among the genes encoding exopolysaccharide synthase. The inhibition of epsAB may be achieved by inhibiting the expression of exopolysaccharide synthase, inhibiting the enzymatic activity of exopolysaccharide synthase, etc. For example, those skilled in the art may select an appropriate method to inhibit exopolysaccharide synthase, such as deleting epsAB from the gene encoding exopolysaccharide synthase, causing a mutation in the gene (mutations such as mutating, substitution or deletion of some bases or introduction of some bases to inhibit normal gene expression), regulating gene expression during transcription or translation, etc.

### Inhibition of a pathway that converts glycerophosphate to glycerol

Glycerophosphatase regulates the conversion of glycerophosphate to glycerol. The pathway that converts glycerophosphate to glycerol may be inhibited by inhibiting the glycerophosphatase. The inhibition of the glycerophosphatase may be achieved by inhibiting the expression of glycerophosphatase, inhibiting the enzyme activity of glycerophosphatase, etc. For example, those skilled in the art may select an appropriate method to inhibit glycerophosphatase, such as deleting dgp, a gene encoding glycerophosphatase, causing a mutation in the gene (mutations such as mutating, substitution, or deletion of some bases or introduction of some bases to inhibit normal gene expression), regulating gene expression in the transcription process or translation process, etc.

### Inhibition of a pathway that converts acetoin to (2R,3 S)-butanediol

(2R,3S)-butanediol dehydrogenase regulates the conversion of acetoin to (2R,3S)-butanediol. The pathway that converts acetoin to (2R,3S)-butanediol may be inhibited by inhibiting the (2R,3S)-butanediol dehydrogenase. The inhibition of the (2R,3S)-butanediol dehydrogenase may be achieved by the inhibition of the expression of (2R,3S)-butanediol dehydrogenase, the inhibition of enzyme activity of (2R,3S)-butanediol dehydrogenase, etc. For example, those person skilled in the art may select an appropriate method to inhibit glycerophosphatase, such as deleting budC, a gene encoding (2R,3S)-butanediol dehydrogenase, causing a mutation in the gene (mutations such as mutating, substitution, or deletion of some bases or introduction of some bases to inhibit normal gene expression), regulating gene expression during the transcription or translation process.

### Inhibition of a pathway that converts acetoin to (2R,3R)-butanediol

(2R,3R)-butanediol dehydrogenase regulates the conversion of acetoin to (2R,3R)-butanediol. The pathway that converts acetoin to (2R,3R)-butanediol may be inhibited by inhibiting the (2R,3S)-butanediol dehydrogenase. The inhibition of (2R,3R)-butanediol dehydrogenase may be achieved by inhibition of expression of (2R,3R)-butanediol dehydrogenase, inhibition of enzyme activity of (2R,3S)-butanediol dehydrogenase, etc. For example, those person skilled in the art may select an appropriate method to inhibit glycerophosphatase, such as deleting gdh, a gene encoding (2R,3R)-butanediol dehydrogenase, causing a mutation in the gene (mutations such as mutating, substitution, or deletion of some bases or introduction of some bases to inhibit normal gene expression), regulating gene expression during the transcription or translation process, etc.

### Inhibition of a pathway that converts glutamic acid to polyglutamic acid

Polyglutamate synthase regulates the conversion of glutamic acid to polyglutamic acid. The pathway that converts glutamic acid to polyglutamic acid may be inhibited by inhibiting the polyglutamate synthase. The inhibition of polyglutamate synthase may be achieved by inhibiting the expression of polyglutamate synthase, inhibiting the enzyme activity of polyglutamate synthase, etc. For example, those person skilled in the art may select an appropriate method to inhibit polyglutamate synthase, such as deleting pgsBCAE, a gene encoding polyglutamate synthase, causing a mutation in the gene (mutations such as mutating, substitution, or deletion of some bases or introduction of some bases to inhibit normal gene expression), regulating gene expression during the transcription or translation process, etc.

### Production method of polyol and production method of exopolymer

The present invention relates to a method for producing polyol comprising the steps of preparing a recombinant microorganism; and culturing the recombinant microorganism in a medium containing a carbon source.

The present invention also relates to a method for producing exopolymer comprising the steps of preparing a recombinant microorganism; and culturing the recombinant microorganism in a medium containing a carbon source.

The present invention also relates to a method for simultaneous producing a polyol and a exopolymer, comprising the steps of preparing a recombinant microorganism; and culturing the recombinant microorganism in a medium containing a carbon source.

The culturing is performed under aerobic conditions, preferably under microaerobic conditions. For example, the culturing is performed while supplying oxygen, i.e., air, and as a specific example, the culturing may be performed through stirring, but is not limited thereto. It is apparent that the recombinant microorganism of the present invention may be cultured in a complex medium, and the type of complex medium is not particularly limited, and those skilled in the art may appropriately select and use a complex medium that is generally commercially available.

The carbon source may include one or more selected from the group consisting of sucrose, glucose, and glutamic acid. In one example, the carbon source may include two or more selected from the group consisting of sucrose, glucose, and glutamic acid.

In one example, glucose may be used as a carbon source for exopolysaccharide production. In one example, glucose and glutamic acid may be used simultaneously as carbon sources to produce polyglutamic acid. In one example, sucrose may be used as a carbon source for levan production.

The culturing may be performed at 25 to 55 °C. Those skilled in the art may appropriately select the culture temperature depending on the type of polyol or exopolymer desired. For example, in order to produce exopolysaccharide with high selectivity, the culturing may be performed at a temperature of 25°C or higher and 37°C or lower. For example, in order to produce polyglutamic acid with high selectivity, the culturing may be performed at a temperature of 37°C or higher and 55°C or lower. Levan may be produced with high selectivity over the entire temperature range of 25 to 55 °C.

The method for producing polyol may further comprise the step of recovering the polyol from the culture medium obtained by culturing the recombinant microorganism. In addition, the method for producing exopolymer may further comprise the step of recovering polyol from the culture medium obtained by culturing the recombinant microorganism.

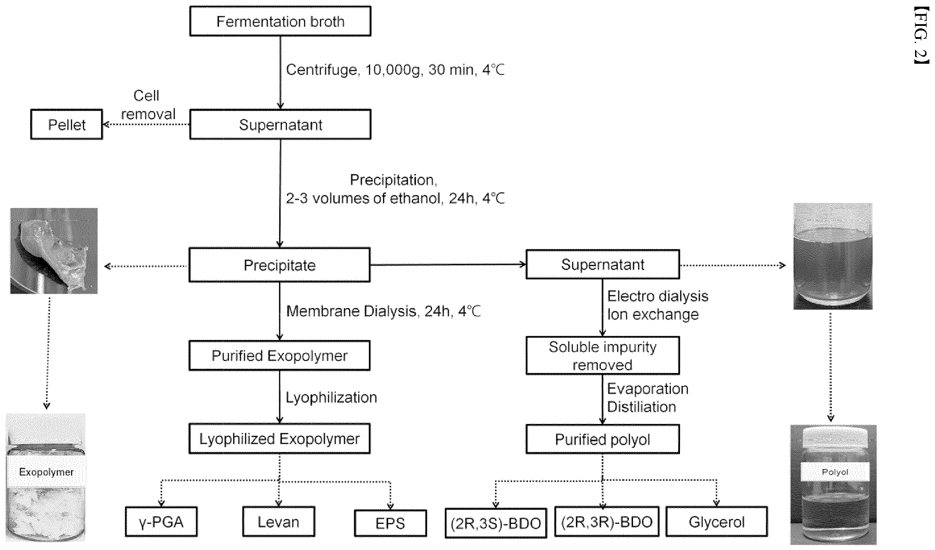

In addition, the present invention may simultaneously recover polyol and exopolymer from the culture medium as shown in FIG. 2.

To recover exopolymer, cells are removed through centrifugation of the culture medium, and two to three times as much ethanol are added to the supernatant and precipitation is formed to obtain solid crude exopolymer. Then, high purity viscous polymers may be obtained through additional subsequent processes such as washing, membrane dialysis, and lyophilization. In addition, by utilizing the high boiling point (above 180°C) of polyol such as 2,3-butanediol and glycerol, high purity polyol products may be obtained through a distillation process. Therefore, two different substances may be recovered simultaneously by recovering the exopolymer through precipitation from the fermentation broth from which cells have been removed and recovering the polyol from the remaining supernatant through a distillation process.

In an embodiment, cells are removed from the culture medium by centrifugation (3,500 rpm, 10 minutes), and two to three times more volume of ethanol is added to the supernatant from which cells have been removed to precipitate exopolymer. The precipitated exopolymer is recovered in the form of powder through membrane dialysis, lyphylization, etc., and polyol may be recovered through distillation from the residual liquid from which the exopolymer has been removed using the characteristic of high boiling point.

### [Mode for Invention]

The advantages and features of the present invention and a method of achieving them will become more readily apparent from the embodiments to be described in detail. However, the present invention is not limited to the embodiments disclosed herein, and various changes may be made thereto. The embodiments disclosed herein are provided only to inform one of ordinary skilled in the art of the category of the present disclosure. The present disclosure is defined only by the appended claims.

### <Materials and Method>

Bacillus licheniformis GSC4071 (KCTC 14485BP), a wild-type strain isolated from a soil sample near Daejeon, was prepared. In the following experimental examples, viscous polymer is a general term for solids obtained by removing cells after culturing and precipitating them with ethanol. Specifically, it includes polyglutamic acid and levan, and is also a general term for several types of exopolymers, including other viscous polymers.

The concentration (g/L), yield (g/g), and selectivity (%) of the viscous polymer or polyol were calculated as follows.
- The concentration of viscous polymer or polyol (g/L): Amount of viscous polymer or polyol produced per unit volume
- The yield of viscous polymer or polyol (g/g): (Production of viscous polymer or polyol (g)/carbon source (g)) × 100
- Selectivity of viscous polymer or polyol (%): (production of specific viscous polymer or polyol (g)/total production of viscous polymer or polyol (g)) × 100

### <Experimental Example 1>

Recombinant strains were prepared using Bacillus licheniformis GSC4071.

### <1-1> Construction of recombinant B. licheniformis with deletion of epsAB, dgp, budC, gdh, and pgsBCAE

### Production of DNA fragments containing homologous regions of target genes

To inactivate the target gene of Bacillus licheniformis, the bacterial recombination mechanism was used, and the homologous region of the gene to be removed was amplified by PCR. Thereafter, the corresponding DNA fragment containing the homologous region is delivered to the bacteria, and then the target gene is removed through a recombination mechanism using recombinase, between the homologous region of the gene in the DNA fragment and the gene in the chromosome of Bacillus licheniformis.

First, in order to clone the homologous region of the EPS synthase of Bacillus licheniformis, the homologous region 1 (SEQ ID NO: 2) of the target gene, epsAB (SEQ ID NO: 1), was amplified by PCR using the primers of SEQ ID NOs: 3 and 4. In addition, homologous region 2 of epsAB (SEQ ID NO: 5) was amplified by PCR using primers of SEQ ID NOs: 6 and 7. Thereafter, homology regions 1 and 2 were simultaneously amplified by PCR as a template to complete a DNA fragment (SEQ ID NO: 8) containing homology regions 1 and 2.

Meanwhile, in order to clone the homologous region of polyglutamate synthase of Bacillus licheniformis, homologous region 1 (SEQ ID NO: 10) of the target gene, pgsBCAE (SEQ ID NO: 9) was amplified by PCR using primers of SEQ ID NOs: 11 and 12. amplified. In addition, homologous region 2 (SEQ ID NO: 13) was amplified by PCR using primers of SEQ ID NOs: 14 and 15. Thereafter, homology regions 1 and 2 were simultaneously amplified by PCR as a template to complete a DNA fragment (SEQ ID NO: 16) containing homology regions 1 and 2.

Meanwhile, in order to clone the homologous region of the glycerophosphatase of Bacillus licheniformis, homologous region 1 (SEQ ID NO: 18) of the target gene, dgp (SEQ ID NO: 17) was amplified by PCR using primers of SEQ ID NOs: 19 and 20. In addition, homologous region 2 (SEQ ID NO: 21) was amplified by PCR using primers of SEQ ID NOs: 22 and 23. Thereafter, homology regions 1 and 2 were simultaneously amplified by PCR as a template to complete a DNA fragment (SEQ ID NO: 24) containing homology regions 1 and 2.

Meanwhile, in order to clone the homologous region of (2R,3S)-butanediol dehydrogenase of Bacillus licheniformis, homologous region 1 (SEQ ID NO: 26) of the target gene, budC (SEQ ID NO: 25) was amplified by PCR using primers of SEQ ID NOs: 27 and 28. In addition, homologous region 2 (SEQ ID NO: 29) was amplified by PCR using primers of SEQ ID NOs: 30 and 31. Thereafter, homology regions 1 and 2 were simultaneously amplified by PCR as a template to complete a DNA fragment (SEQ ID NO: 32) containing homology regions 1 and 2.

Meanwhile, in order to clone the homologous region of (2R, 3R)-butanediol dehydrogenase of Bacillus licheniformis, homologous region 1 (SEQ ID NO: 34) of the target gene gdh (SEQ ID NO: 33) was amplified by PCR using primers SEQ ID NOs: 35 and 36. In addition, homologous region 2 (SEQ ID NO: 37) was amplified by PCR using primers of SEQ ID NOs: 38 and 39. Thereafter, homology regions 1 and 2 were simultaneously amplified by PCR as a template to complete a DNA fragment (SEQ ID NO: 40) containing homology regions 1 and 2 (Table 1).

**[Table 1]**

| SEQ ID NO | sequence |
|---|---|
| 1 | atgaaagaaaatattgattttagagaactgattgcaatcttgcgaaaaagaacggttcttattctcgttttgacaa taggtgtaacattgacgaccggaatcattcagttctatgtgctgacacctgtctatcaggcatcgacgcagatctt ggtgcatcaagtaggggagaaaaaggggagcgccacatacagcgatattcaaatcaatcttcaatacacacggaca ttccaagcgcttttgaaaaacccggtgattttggagcaagtcaagagagagcttgatttaccttactctgcgggcc ggttgggtgaaaaaattgcaacgagcagtgaaagcgaatctgagattataaatatttcggtccaagatgaaaatca gaaacgggcggccgatatagcgaacactttaactgcggtgctcaaaaaagagattaagcaaattatgaacaccgat cgggtaaccgtcctgtcaaaagccgaaatagtcgattcgccgacacctgtcagaccgaattacaaaatgaatattt tgctggcattcggcgccgcattaatgaccggaatcgctttggcgttctttttggactttatcgatgatacggttgc aagaccgtctcaagtcgaaaaggaagcgggattcatttatttgggaagtattgagcaaatgaagcataaaaaaagt ctgtttcgcggggaccccgatatgaatatccgcgtaaaagcaggaaggagtgagccgcttgggtattagaaaaaaa cgctctcgcaaatatcaatcggcgcttgtcgcattgcatcagccgaacacgccgatcgtcgaacagtatcggacga tcaggacgaacattgagttttcatcatttgagaagccgttcaagtcattgctcattacatcgggcctgccgggaga aggcaaatcattctcggcttcaaacttggcgatcgtattttcgcaacaggaaaaaaaggtccttttgatcgacgca gatttaagaaagccgacgatccataaaatttttgagctcgataaccattcaggcgtcacaaatgtattaatgaaaa aatcgacgctggaaaatgtcgtccagcaaagccaggcggaaaatctccatgtgctgacaagcggtccgattcctcc gaatccgtccgagcttttgtcgtcgcaggcgatggaggacctgcttgcggaagcgtacgaccaatacgatttagtc atccttgattcgccgccgcttttgccggtcgcagacgcgcaaatattggcgaatcaggtggatggaagcattcttg tcatcctcagcggaaaaacaaagcttgataacgcgatcaagtctcgggacgcgctgaattcttccaaaagcgaact gctcggcgccgtgctgaacgggcggaaagtgaagaaagcgcgccaatataattacgcaaccatgtaa |
| 2 | gtacaagatgggttctccactcatcttcctgctcagtccggcgataaaggatcacttcggtatcaaacaattcctt taattcgactttcagcgcccgggagatttttttcaggaattcgactgacggatttctgtgaacgccgcgttcaatt ttgcttaaatatgattttgatacgcctgctatatcagcaagctggttaatggagtagccttttctctttctgtaca ttcggatgactcttccgatcatactgaatcctccccttcccttgccattcatataaaacgctttcctgttcattat aagaaataatttgttctttataaaattcaaaaatttaagatatcgacagaaaataaaagaaaacgaacgaatttgt tctctaaaaagaactatatagctaaattcagcatttttcaattcaaattttttcttttataatccaatcattaaca aacggcattcctgaggaggcagtcaagc |
| 3 | agacagggccaacgaggccgtacaagatgggttctccac |
| 4 | agccgcttcataagagcttgactgcctcctcag |
| 5 | tcttatgaagcggctgcagccgctttaggtgatgtctccttgcctttaccgatggaggcactcggctctaccgtgg gcagactacctgtctgccttagacgcaagtcgtcgatggcggggcgtgaggacgggttaaatgcccatctttattt aatttacgtgctcggtttaaatttgaaaatcgcccgtatctttttagctcatgggattttgccctgaaagatccta tcagctagaaagatacattaggaggtaggaaattgacatatcggagaaggctttccattattaccgcactcgattc |
| | gtacttggttttgctgtccatctttatcggatatcagctgattttgccatcatatgatttatacccttcggaaatg ctgctgatgacttcactgatactgcttggcgctcagcatttattcgcccattgcttccacctttataaaaaagtat gggagtatgcaagcatcggtgaattgtatgtgctgcttaaatcgattacattgtcccatcttgtgacggcggccct cgagctgtttttctttcaaaacgttccggttcgtcttttatgtttaagctggctgttccagctcattttgatcggc ggatcgcggatgatgtggcgcatcatcagggaacaggtgaacaaagaaagcaaaggatctctaagggcgcttatca tcggagcgggctctgccggcagtttgattgcaaaacagcttgtg |
| 6 | aggaggcagtcaagctcttatgaagcggctgc |
| 7 | agacagggccttattggccgtcacaagatgggacaatg |
| 8 | gtacaagatgggttctccactcatcttcctgctcagtccggcgataaaggatcacttcggtatcaaacaattcctt taattcgactttcagcgcccgggagatttttttcaggaattcgactgacggatttctgtgaacgccgcgttcaatt ttgcttaaatatgattttgatacgcctgctatatcagcaagctggttaatggagtagccttttctctttctgtaca ttcggatgactcttccgatcatactgaatcctccccttcccttgccattcatataaaacgctttcctgttcattat aagaaataatttgttctttataaaattcaaaaatttaagatatcgacagaaaataaaagaaaacgaacgaatttgt tctctaaaaagaactatatagctaaattcagcatttttcaattcaaattttttcttttataatccaatcattaaca aacggcattcctgaggaggcagtcaagctcttatgaagcggctgcagccgctttaggtgatgtctccttgccttta ccgatggaggcactcggctctaccgtgggcagactacctgtctgccttagacgcaagtcgtcgatggcggggcgtg aggacgggttaaatgcccatctttatttaatttacgtgctcggtttaaatttgaaaatcgcccgtatctttttagc tcatgggattttgccctgaaagatcctatcagctagaaagatacattaggaggtaggaaattgacatatcggagaa ggctttccattattaccgcactcgattcgtacttggttttgctgtccatctttatcggatatcagctgattttgcc atcatatgatttatacccttcggaaatgctgctgatgacttcactgatactgcttggcgctcagcatttattcgcc cattgcttccacctttataaaaaagtatgggagtatgcaagcatcggtgaattgtatgtgctgcttaaatcgatta cattgtcccatcttgtgacggcggccctcgagctgtttttctttcaaaacgttccggttcgtcttttatgtttaag ctggctgttccagctcattttgatcggcggatcgcggatgatgtggcgcatcatcagggaacaggtgaacaaagaa agcaaaggatctctaagggcgcttatcatcggagcgggctctgccggcagtttgattgcaaaacagcttgtg |
| 9 | atgtgggtaatgctattagcctgtgtgatcgttgttgggatcggcatttatgaaaaaaggcgccaccagcaaaata tcgatgcgctgcctgtccgagtgaacatcaacggtatacgcggaaagtccacggtgacaagattaacaacagggat attaatcgaagcaggctacaaaacagtaggaaaaacaaccgggacagacgcaaggatgatttattgggacacaccg gaagagaagccgatcaaaagaaagccgcaagggccgaatatcggagagcagaaggaggttatgaaagaaacggtgg aaagaggggccaatgcgattgtcagtgagtgcatggccgttaatcctgattaccaaatcatctttcaggaagaatt gcttcaggctaatatcggcgtgatcgtgaacgtgctggaggatcacatggatgtgatgggaccgactttggatgaa atcgcagaagcattcacagcaaccattccttataatggacatttggttattactgatagtgagtataccgatttct ttaagcaaattgcaaaagaaaggaacacaaaagtcatcgtcgcagacaattctaaaataacagatgaatacctcag acagtttgagtacatggtattccctgataatgcgtctcttgcgctcggtgtagctcaagcgttgggcattgacgaa gaaaccgcctttaaaggcatgctgaatgcgccgcctgatccgggagccatgagaattctgccgctgatgaacgcca agaatcccggacatttcgtcaacggttttgcggccaatgacgcagcttccactttaaacatttggaagcgtgtaaa agaaataggctatcctacggatcagccgatcgtcattatgaactgccgcgccgacagggtagacagaacacagcag tttgcggaagatgtccttccttatattgaagcaagtgaacttgtgctgattggagaaacaacagagccgatcgtca aagcatatgaagcaggcaaaattcctgcggacaagctgtttgattttgagcacaaatcaacggaagaaatcatgtt catgctgaaaaacaagcttgagggccgcgttatttacggagtcggaaatatccacggagcagcggagcctctcatt |
| | gaaaaaatacaagattacaagattaagcagctcgttagctaggaggaagaaaacacaatgtttggatcagatttat atatcgccctcattttaggagtcttactcagtttgatttttgcggagaaaacgggaattgtaccagccggcctcgt cgtaccgggttatttgggacttgtcttcaatcagccgattttcatgctgctcgttctttttgtcagtttgctgacg tatgtcatcgtgaaattcggactttccaaaattatgattctatacggacgcagaaaattcgcagcaatgctgatta cgggaattcttttgaaaatcggttttgattttatatatccggtgatgccgtttgagattgccgaattcaggggaat cggaatcatcgtgccggggctgatcgccaataccattcaaagacagggattaacgattacgcttggaagtacgctt ttattgagcggagcaacattcgtcattatgtatgcttactatctaatctaaagttaggtgtgtcaaatcgatgaaa aaacaactgaactttcaggaaaaactgctgaagttgacgaagcaggagaaaaagaaaacaaacaagcacgtcttta tcgtattgcccgttattttctgtttaatgtttgtctttacttgggtcggaagcgccaaaactccttcgcaaatgga caaaaaagaagatgccaagcttacagctacttttgttggcgatatcatgatgggaagaaacgtagaaaaagtgaca aacttgcacggttcggaaagtgtcttcaaaaatgtgaagccgtactttaatgtgtcagattttatcacaggaaact ttgaaaaccctgtaaccaatgcaaaggactatcaagaggcagaaaagaacatccatctgcaaacgaatcaagaatc agtcgaaacattgaaaaagctgaacttcagcgtactgaattttgccaacaaccatgcgatggactacggggaagac ggtttgaaggatacgctcaataaattttcaaatgagaatctggagcttgtcggagcaggaaataatcttgaagacg cgaaacagcacgtatcctatcagaatgtgaacggcgtaaaaattgcaacgctcggttttacagacgtctacacaaa gaactttacagccaaaaagaacagaggcggagtgctgccgctcagtccgaaaatctttattccaatgattgcggaa gcatcgaaaaaagcggatcttgtccttgtccatgtgcactggggacaagaatatgacaatgaaccgaacgacagac agaaggatctggccaaggcgattgcagatgccggagcagatgtcatcatcggcgctcatccccatgttctcgaacc gatcgaagtgtataacggtactgtgattttctacagcctcggcaactttgtatttgatcagggctggtcaagaaca cgggacagcgcgcttgtacaataccatttaatgaatgacggcaaagggcgttttgaggtaacgcctctcaacattc gcgaagcaacgccgacgcctttaggcaagagcgacttcttaaaacgaaaagcgatcttccgtcaattgacaaaagg aacaaacctcgactggaaagaagagaacggaaaattaacgtttgaagtcgatcatgcggacaagctgaaaaaataa taaaaacggagtggtgaacaaatgaaatttgtcagagccatttggccgttcgtaggtttagttttgatcattgcat tcatgtctgctttcaagtattctgatgaattgtcaaacgatgaaaaagctaaaatctctacggagatccaaaaagt gaatcagcaggatcaaacaacagaaaacaagcaataa |
| 10 | aagccttctcctctctatttgtgtaaccgtttacataaagattatcacgatccatttaaaatcacaataaaaaaat ggatgaacactgaaattttagaccgggctgggatatcgagacatatataggggcgtttaatggcgaatacagtaac aatgagaatagtaagaaaaattaaaaatgttaaagtttgatgaattatcattgaaaaaaattaatggctttttaaa tcctaggattttaacctaaaatctgaagaaataaggtggatcgaacgactcacaaaatatttggatttgtcaatga atcccgctttatgctaaaagagattttcattttttgatagatggtctgattgtcataggacggatttgttttgaag agggaacattggtgactttttaacctgttcgaaaagagcgaaaatactaaaagaaaagagagatcccggctgacag cccatttaaaggggattgcggacgggggaaaaaagagatcctgaatccatccttcaacctttcatctgaaataggg agaaaagtacaaaaatcataatgtcgaattttgaaagcgcatacttaaaacgctgacaaaaatctgataggaatta agaactttcgatttccaaaaatatcaataaaaagataggcattaatgactcgggcgaggtgatctttgtcacggaa aatttcgtcgtcttctgttacataatgccgattgtgatttcatagtgaacctatatactgatgaatgaatttacat atcagattccaagaaggagatgtagacaaaca |
| 11 | agacagggccaacgaggccaagccttctcctctctatttgtgtaac |
| 12 | cggcactctcttttgcactgtgtttgtctacatctccttc |
| 13 | cagtgcaaaagagagtgccgccggcgctctcttttttgtgtgctgatcccggttataaaagacctgtgaaaagcga |
| | ccggtttgaaagggaaacacgacaattttcttaaccggtcagtgtataaagttttatagaaaatcaggaggatata tacatggttttggggttcatgtttattgtattcttttgaagggaataaaaactgacaaatttcgactgaagcaaaa tttgaaaatgcatcaccttaccaattcgggatgggaaccgcacctcatgttcatgacctctttagaatatttccct tcatctttttaatctgcgcttaggtgaaaaagctgatcatgctgtgctgagcgtttcttctcgctatgacgctgct gtacatgcaaaaaaagtcctttaaatatcccagttgaatgacgatgaaagaggaaagaagaggaggaacagatcaa ttgataaaaaaagcggcaaacaaaaagttggttttgttttgtggaattgcggtgctttggatgtctttatttttaa cgaatcataatgatgtacgcgccgatacgatcggcgagaaaatagcggaaactgccagacagcttgagggtgcgaa atacagctacggcggagagaagccgaaaacggggtttgactcgtcaggctttgtgcaatatgtgtttcaatcgctc gatattacgcttccgagaacggtaaaggaacaatcgactcttgggagcagt |
| 14 | gaaggagatgtagacaaacacagtgcaaaagagagtgccg |
| 15 | agacagggccttattggccactgctcccaagagtcgattgttc |
| 16 | aagccttctcctctctatttgtgtaaccgtttacataaagattatcacgatccatttaaaatcacaataaaaaaat ggatgaacactgaaattttagaccgggctgggatatcgagacatatataggggcgtttaatggcgaatacagtaac aatgagaatagtaagaaaaattaaaaatgttaaagtttgatgaattatcattgaaaaaaattaatggctttttaaa tcctaggattttaacctaaaatctgaagaaataaggtggatcgaacgactcacaaaatatttggatttgtcaatga atcccgctttatgctaaaagagattttcattttttgatagatggtctgattgtcataggacggatttgttttgaag agggaacattggtgactttttaacctgttcgaaaagagcgaaaatactaaaagaaaagagagatcccggctgacag cccatttaaaggggattgcggacgggggaaaaaagagatcctgaatccatccttcaacctttcatctgaaataggg agaaaagtacaaaaatcataatgtcgaattttgaaagcgcatacttaaaacgctgacaaaaatctgataggaatta agaactttcgatttccaaaaatatcaataaaaagataggcattaatgactcgggcgaggtgatctttgtcacggaa aatttcgtcgtcttctgttacataatgccgattgtgatttcatagtgaacctatatactgatgaatgaatttacat atcagattccaagaaggagatgtagacaaacacagtgcaaaagagagtgccgccggcgctctcttttttgtgtgct gatcccggttataaaagacctgtgaaaagcgaccggtttgaaagggaaacacgacaattttcttaaccggtcagtg tataaagttttatagaaaatcaggaggatatatacatggttttggggttcatgtttattgtattcttttgaaggga ataaaaactgacaaatttcgactgaagcaaaatttgaaaatgcatcaccttaccaattcgggatgggaaccgcacc tcatgttcatgacctctttagaatatttcccttcatctttttaatctgcgcttaggtgaaaaagctgatcatgctg tgctgagcgtttcttctcgctatgacgctgctgtacatgcaaaaaaagtcctttaaatatcccagttgaatgacga tgaaagaggaaagaagaggaggaacagatcaattgataaaaaaagcggcaaacaaaaagttggttttgttttgtgg aattgcggtgctttggatgtctttatttttaacgaatcataatgatgtacgcgccgatacgatcggcgagaaaata gcggaaactgccagacagcttgagggtgcgaaatacagctacggcggagagaagccgaaaacggggtttgactcgt caggctttgtgcaatatgtgtttcaatcgctcgatattacgcttccgagaacggtaaaggaacaatcgactcttgg gagcagt |
| 1.7 | atgaagaaatgggcatttgtatcagactttgatgggacgatttccaaacaggatttttactggatggtcattgata aatattttcccgaaggccgtgaattgttcaagaagtggaagtccggagaattaaaagatatcgaatttttgggaac cgtttttgcttcgattaatcaaagcgaacaaaaaatcattgatgacatccattccataccgattgatgaatatgtg cctgacttcattcagcatgtccagaaaagcggcggcgacttctacattttaagcgccggcacggattattacattc attatattttaaagaaatacgggattacagacgttgaggtctattcaaataaaggcttttttaaagaagacaatgt tcatatggacattgatgagaatcattggcattactctgagcgctatgggattgataaatcaaaggtcattcaaaag ctgaaagaagagtatgagacggtttattttgcaggcgacagtgaaccggattcgcacccggctaaatttgcggatg |
| | ttacgtttgcaaaggatgcgctccaggatttgctgcgtcagcagggggtgccatttgtagccgttgaaacatttga agacattgaacaatatcttaaagaaaaagggcggatcgtctaa |
| 18 | ttacgtttgcaaaggatgcgctccaggatttgctgcgtcagcagggggtgccatttgtagccgttgaaacatttga agacattgaacaatatcttaaagaaaaagggcggatcgtctaa cctaagtgataatgattcgcattatcaatgtaaatatagcagatcaatccctctctcgtctatcactttttttgct aagtatgattagtgtggttttggttaatttgttactataaacagaaaaaggactgatgagagatggcagaaacgaa cccggtgatcaaaaaattgcagttcaaggataatggacagcctgtactgattgtgaatccgcctgaggcatacagc ggcgtcatcgctgaatttcaaggccctgttcatcatcacgccgaatcggaccaatatgattttgttcaggtattcg cagcgaccaacgcaaagctgcaggctttagccaaggaggctgaacgtcatttggcggaagacggtctgttttggct ttgctacccgaaaaaatcatcgaaagtctacaaaggatcggactgcagccgcgatacagtagccggactgcttgca gaccagggctatgaaccggttcgccaaattgcaattgatgaagactggtctgcgctcagatttcgcaaggcggaaa acatcaaaacgatgaagcggaaatttgccgtaacagagaaaggtaagcagcgtacagatcaagaatgattatatac gcctcctttcagggggacgaatattcagcttgtgaacgaaagtacacaaaaaagaaataatattatgttcaatgat tcacaaactttttgtataatgaatctaatcttttaaaataccatagaaaagaggtatgcagc |
| 19 | agacagggccaacgaggcccctaagtgataagtattcgcattatc |
| 20 | cggcattagacgatcgctgcatacctcttttctatgg |
| 21 | gatcgtctaatgccgcgccaggttaccagcatagccatccccgcggttttggacattagtgagggcgtgcttggcc ggttggatgagatattgagaaaacatcagtttgacaaggcgatcatgttttttgacgatttttcctatcagcagta tgagcattcactgaaaaccgcatttcaacatgttaaagttgaagccgttctgctgccgtccaatctggacatacag gagcttttgaagcgggcattttcgatcgggaattgcgatgtcattattgccatgggcggcgggtatgttgtggact gcggaaaatacatcgccttttccaagcggaccccgttcatcagcattccgacgtcggcatccaatgacgggtttgc gagcagcaattgctcgcttcaggtcgaaggaaaaaaaacaaccgttccggcaagggttccctacggaatcatagct gatttgagcattattcaaaaagcgccggattgctttattttggcggggatcggagatttgatgtccaatattacgg ccctttatgattgggagtttgaggagcggcacggggtcggcgatgtaaatgcgtttgcgtcgatgctcagcaagaa agcggtcaacagctttgtccgtacgccgatgcaggatattaaacagccgatcttcttaaaagagcttgtcagttct ttgacaatgggcggcatcgctacggaaatcagcggaaac |
| 22 | aaagaggtatgcagcgatcgtctaatgccgcgc |
| 23 | agacagggccttattggccgtcaaagaactgacaagctcttttaag |
| 24 | cctaagtgataatgattcgcattatcaatgtaaatatagcagatcaatccctctctcgtctatcactttttttgct aagtatgattagtgtggttttggttaatttgttactataaacagaaaaaggactgatgagagatggcagaaacgaa cccggtgatcaaaaaattgcagttcaaggataatggacagcctgtactgattgtgaatccgcctgaggcatacagc ggcgtcatcgctgaatttcaaggccctgttcatcatcacgccgaatcggaccaatatgattttgttcaggtattcg cagcgaccaacgcaaagctgcaggctttagccaaggaggctgaacgtcatttggcggaagacggtctgttttggct ttgctacccgaaaaaatcatcgaaagtctacaaaggatcggactgcagccgcgatacagtagccggactgcttgca gaccagggctatgaaccggttcgccaaattgcaattgatgaagactggtctgcgctcagatttcgcaaggcggaaa acatcaaaacgatgaagcggaaatttgccgtaacagagaaaggtaagcagcgtacagatcaagaatgattatatac gcctcctttcagggggacgaatattcagcttgtgaacgaaagtacacaaaaaagaaataatattatgttcaatgat tcacaaactttttgtataatgaatctaatcttttaaaataccatagaaaagaggtatgcagcgatcgtctaatgcc gcgccaggttaccagcatagccatccccgcggttttggacattagtgagggcgtgcttggccggttggatgagata ttgagaaaacatcagtttgacaaggcgatcatgttttttgacgatttttcctatcagcagtatgagcattcactga |
| | aaaccgcatttcaacatgttaaagttgaagccgttctgctgccgtccaatctggacatacaggagcttttgaagcg ggcattttcgatcgggaattgcgatgtcattattgccatgggcggcgggtatgttgtggactgcggaaaatacatc gccttttccaagcggaccccgttcatcagcattccgacgtcggcatccaatgacgggtttgcgagcagcaattgct cgcttcaggtcgaaggaaaaaaaacaaccgttccggcaagggttccctacggaatcatagctgatttgagcattat tcaaaaagcgccggattgctttattttggcggggatcggagatttgatgtccaatattacggccctttatgattgg gagtttgaggagcggcacggggtcggcgatgtaaatgcgtttgcgtcgatgctcagcaagaaagcggtcaacagct ttgtccgtacgccgatgcaggatattaaacagccgatcttcttaaaagagcttgtcagttctttgacaatgggcgg catcgctacggaaatcagcggaaac |
| 25 | atgagtaaagtatctggaaaaattgcttttgttactggcggcggtcaaggaattggagaagcaatctgcaaacgat tggcagaggacggattcgcagttgcagttgccgattataatgtagaaactgcaacacaagttgctgaggacatcaa taagcttaacggcaaagcaattgcggttaaagtggatgttgctgatcgcgatgatgtttttaaagctgtcgatgaa acagtaaaacgtcttggcggtcttgatgtggtgattaataatgcgggtcttggaccaaccacccctattgaaagca ttacatatgaagattatcggaaagtctatgatgttaacgttggcggtacttattggggaatacaagcagctgtaaa agcctttaaagaacttggacacggcgggaaaatcattaatgcatcttctcaagccggccaagtcggcaacccgggc ttagcggtttacggaggaacaaagttcgctgttcgcgggattacccaaactgcggcaaaagatctagctgaattag gtattactgtaaacgccttttgtccgggtatcgttaaaactcctatgatgatggggattgcacagcaaaccgctga tgaagcaggcaagccgtttgaatggggcatggaacaattcgctaaaaatattgcattaaaacgcttatccgagccg gaagatgtagcagcatgcgtttcttaccttgcagggccagattcagattatatgactggtcaagctcttatcattg atggcggaatggtatttaattaa |
| 26 | actccatgaactgacagtcggaaaatatggtgaagaaaaggcgaaatcattatggaattcactcggtttcggagct gaatccgtccttctcttaatcttagctgccgcttccggtcaagcgttcatgatgaacaggcggaaagagcatctga aggcatttatgctcgcggtgttctatcactcttcttatttcgataaagcgcatgttttaaaaccataaccatacaa aaaaagcgggaggttttaacatgttatttatcataagaatggtgattattggcctattttcattgacagcaatcaa tctgtttgtttttcaaggaattgaattcatgcacgcgattaccgatcttttcaatcgccaggacagctgaatttga agcgatttgaaaaagctagacaataaaatttcatgaagcagtttgtttggcatgaatccggcattgtgcgggttca tgccttttcatttggtcttcatccgtttagcgatgactgaatcgctgttttatagcagaacatcccttttatagtt ttacatacagttcttcctccctctttcaaaaactattaaattttttcgaaaaattttaaacatattttcaacaaat gccgcggctttacttcaaatctacaaatataagatataatattcctgtaccaattaaataccaattacttatagaa agaagatgtttatatgagtaaagtatctggaaaaattgcttttgttactggcggcggtcaaggaatt |
| 27 | agacagggccaacgaggccactccatgaactgacagtcg |
| 28 | catcaatgataagagaattccttgaccgccgccagtaac |
| 29 | ctcttatcattgatggcggaatggtatttaattaattttaaaattatattctatatggagggaggagttttcccct ccatatttagtattgaataatcagctatatagcatggcttaaaaccgtgtaatacgaaacggccacggcgtccaac cacgccttgatttatggttcacgtttaaaaaacaaagaacgcaggccgttggttcctcccccatcggcaaatcgtg gcgtccatgaatttcgaatcgcatcaatacttagcgtttactgccaaattgtcattctaaacgcccttcttcttga agttttcccaattttttatgattgcccaggtccctttatctgttgaaggacggctccatcatttaggatataacga agcgggacaattgagcggaaatgatacaaacaagaggctaattttaattaaaaaaaggccgaagtgagcaacttcg ccctcaagcaaggcgccgtgaggaaatccatatgtttacgatcttttgaacccctgtttcaaaatgcactagtgcg |
| | aggatttcccctgcgataagcggcacccaagatatcttctttgaattctactccaagcctccataggttttattta cgcgctttttccgtcaactcgtttattttctccatttttttcgatttcctctaaactgcgacctcgagtttcaggg acgcgtgtccggataaatacaactccaagcaaacaaatcacaccgaagattgcaaacacagcttcttgagacatcg aagcggtcataacagg |
| 30 | ggcggtcaaggaattctcttatcattgatggcggaatg |
| 31 | agacagggccttattggcccctgttatgaccgcttcgatg |
| 32 | actccatgaactgacagtcggaaaatatggtgaagaaaaggcgaaatcattatggaattcactcggtttcggagct gaatccgtccttctcttaatcttagctgccgcttccggtcaagcgttcatgatgaacaggcggaaagagcatctga aggcatttatgctcgcggtgttctatcactcttcttatttcgataaagcgcatgttttaaaaccataaccatacaa aaaaagcgggaggttttaacatgttatttatcataagaatggtgattattggcctattttcattgacagcaatcaa tctgtttgtttttcaaggaattgaattcatgcacgcgattaccgatcttttcaatcgccaggacagctgaatttga agcgatttgaaaaagctagacaataaaatttcatgaagcagtttgtttggcatgaatccggcattgtgcgggttca tgccttttcatttggtcttcatccgtttagcgatgactgaatcgctgttttatagcagaacatcccttttatagtt ttacatacagttcttcctccctctttcaaaaactattaaattttttcgaaaaattttaaacatattttcaacaaat gccgcggctttacttcaaatctacaaatataagatataatattcctgtaccaattaaataccaattacttatagaa agaagatgtttatatgagtaaagtatctggaaaaattgcttttgttactggcggcggtcaaggaattctcttatca ttgatggcggaatggtatttaattaattttaaaattatattctatatggagggaggagttttcccctccatattta gtattgaataatcagctatatagcatggcttaaaaccgtgtaatacgaaacggccacggcgtccaaccacgccttg atttatggttcacgtttaaaaaacaaagaacgcaggccgttggttcctcccccatcggcaaatcgtggcgtccatg aatttcgaatcgcatcaatacttagcgtttactgccaaattgtcattctaaacgcccttcttcttgaagttttccc aattttttatgattgcccaggtccctttatctgttgaaggacggctccatcatttaggatataacgaagcgggaca attgagcggaaatgatacaaacaagaggctaattttaattaaaaaaaggccgaagtgagcaacttcgccctcaagc aaggcgccgtgaggaaatccatatgtttacgatcttttgaacccctgtttcaaaatgcactagtgcgaggatttcc cctgcgataagcggcacccaagatatcttctttgaattctactccaagcctccataggttttatttacgcgctttt tccgtcaactcgtttattttctccatttttttcgatttcctctaaactgcgacctcgagtttcagggacgcgtgtc cggataaatacaactccaagcaaacaaatcacaccgaagattgcaaacacagcttcttgagacatcgaagcggtca taacagg |
| 33 | atgtcaaaatcagtaaaatcagtcacatcacctaaaaaatttattacaggaaaacgactgctggagaacttgaacg actacattgaagattttggcgacaacgcatatatcatttgcgatgaattcattttggaacgcgctcaaaaagaagc ggggaattcgattcagaaagccggcaatcaagccgtttttgaaaaattcaattacgaatgcacacaggaagaaatc gatcgcaaccgggagcttgcacgcaatgcaggcgctaatatcatcgttgggatcggaggcggtaaaacgcttgata ccgcaaaagccaccgcttattacgagaagctgccggttgtgattttcccgacaattgcttctacggatgctccatg tacggcccttgccgtcatttataaacacgacggatcgtttgaccgctatctgtttttgccgacgaacccagatgtc gttcttgcggactctgagattttggcatccgcgccgccgcgctttttcgcagccggtatcggtgacgccttggcga cgtattttgaagcgcgcgcctgctttaaagcaaacggcgataacctcgtgctgatgaagccttcaacaactggatt gggacttgcccgtctttgctatgatacgctgttggaaaacggtgtgaaagcgatgcaggcggttaagcacggcgtt tccacacgagcggtcgaagatacaatcgaggcgaccatctatttaagcggcgtcggtgccgaatcaggcggtcttg ccgccgcacacgcgatccacaacggaatgacagccgttccttctctgcacagggctcagcacggcgaaaaagtcac gttcggccttttggcgcagcttgttcttgaaaacgcgccggccgaagaattggagaccgttattgactttatcaaa |
| | ggcgtcggtcttccgttgacattaaaagacctcggagtcgacgaatttgtcgaagaagaatggcgccaagtcgctc aaagcgcttgcgcggaaggcgacacaatgggcaacatgccgttcccagtcacccctgacgacgtctacaatgcgat cgtcgccgccaacgcgattgcagaatcttatcacgattaa |
| 34 | aatgctgaagcgatttccaacaaggcgatccattggggtttttcaaaaagcaaaaaccatcagccggcagatgcgg gtcaagagctgaacaaccttttacagcagtacgacgccttttatttgggcaacacaaaggaaaaaacgatctatct gacctttgataacggctatgaaaacggctacacccctcaggtgctcgatgttctgaaaaaacaaaacgtcaaagcg gccttttttgtgacgggccattttgtcaaagatcagccggagctgatcaagcgaatggccgaggaggggcatatca tcgggaatcattcatatcaccatccggatctgacgacgaaaacaagccgcgtcattcaagaggaattggaatcggt cgatgaggaggtttacaaaatcacaggcgaaaaaaacaacctctacctgagaccgcctcggggcattttcagcgag cgggtgctcgaagaaacgaaaaagctcggctatcaaacggtattctggtctgttgcttttgtcgattggaaaatca atgcccaaaaagggtggcgctatgcgtacgacaatatgatgaaacaggctcaccccggcgccatctatctgcttca caccgtctcgagagacaatgccgaagcgcttgatcaggcgatcaccgacttgaaaaaagaaggttatacatttaaa agcctcgatgacctgatgtttgaaaaatctatgatgcttgagaccctttgaaagaacaatgccccggccgctttgc cggggttttgctttggctgaaaaaattgatgcttcaggctctttttatttcccctagtcaattctatagaataaaa atccattttatatacatatattactagatttaaaaagaaaataggtatatcattgatagtgaaggggaattacc |
| 35 | agacagggccaacgaggccaatgctgaagcgatttccaacaaggcgatc |
| 36 | acggcttttcgtctaggtaattccccttcactatc |
| 37 | tagacgaaaagccgtttccgtgaaggagcggctttctgctttgggttagtatattagtttgtgaatcggcggtctg tccgtgcttttgaatatgaattgtgtggtacgtttcccagagtaaaattatgaatttcttttatagcagtttccca aaaacgagctgtttgactcgtgtgacagccgttcatttcaccactttaatcttcaacctcggctcttcttcaattc cggatgcaaatgcatcctcatttcccggagccgtaaaggtgacgctgtacgtttcctgtgaatgcggcgacagttc cagctcttccggctgaaagtcaaattcatttattatatcaggggtgagctggacgcgttcggtctttttcccgtag ttgttgactttcacctcacagtccatgcttcctgcttccatatcgccctgatagctgcaagcgcttccccttttca tatattccacggcgctatcgccttgtgcattccatttttgaaagaacatcagctgttctgtgaccagcgggtaaaa tactgaaataccgatgataaacaaaaacagcttaaagcggatccacggtactccataataatggcgccacgccata atgaagccggcgatgatcataaacatgcagatcagaaccactaaattccggatgggcagttttaaaaagcccgaaa gcgcgtctgctacagacgcgccttgatgattctcggcgctgatcaacaggccgataacaatcatgatgatgccggc aagccctgtatgtaccttcatttctttccctcctcagttgtagaaatagtcgaacagagaggaaaaaggtttccgg ttttacttgaaaatgtcaaaccatcctttatgccggaaccagaagatcat |
| 38 | tgaaggggaattacctagacgaaaagccgtttccgtgaag |
| 39 | agacagggccttattggccatgatcttctggttccggc |
| 40 | aatgctgaagcgatttccaacaaggcgatccattggggtttttcaaaaagcaaaaaccatcagccggcagatgcgg gtcaagagctgaacaaccttttacagcagtacgacgccttttatttgggcaacacaaaggaaaaaacgatctatct gacctttgataacggctatgaaaacggctacacccctcaggtgctcgatgttctgaaaaaacaaaacgtcaaagcg gccttttttgtgacgggccattttgtcaaagatcagccggagctgatcaagcgaatggccgaggaggggcatatca tcgggaatcattcatatcaccatccggatctgacgacgaaaacaagccgcgtcattcaagaggaattggaatcggt cgatgaggaggtttacaaaatcacaggcgaaaaaaacaacctctacctgagaccgcctcggggcattttcagcgag cgggtgctcgaagaaacgaaaaagctcggctatcaaacggtattctggtctgttgcttttgtcgattggaaaatca |
| | atgcccaaaaagggtggcgctatgcgtacgacaatatgatgaaacaggctcaccccggcgccatctatctgcttca caccgtctcgagagacaatgccgaagcgcttgatcaggcgatcaccgacttgaaaaaagaaggttatacatttaaa agcctcgatgacctgatgtttgaaaaatctatgatgcttgagaccctttgaaagaacaatgccccggccgctttgc cggggttttgctttggctgaaaaaattgatgcttcaggctctttttatttcccctagtcaattctatagaataaaa atccattttatatacatatattactagatttaaaaagaaaataggtatatcattgatagtgaaggggaattaccta gacgaaaagccgtttccgtgaaggagcggctttctgctttgggttagtatattagtttgtgaatcggcggtctgtc cgtgcttttgaatatgaattgtgtggtacgtttcccagagtaaaattatgaatttcttttatagcagtttcccaaa aacgagctgtttgactcgtgtgacagccgttcatttcaccactttaatcttcaacctcggctcttcttcaattccg gatgcaaatgcatcctcatttcccggagccgtaaaggtgacgctgtacgtttcctgtgaatgcggcgacagttcca gctcttccggctgaaagtcaaattcatttattatatcaggggtgagctggacgcgttcggtctttttcccgtagtt gttgactttcacctcacagtccatgcttcctgcttccatatcgccctgatagctgcaagcgcttccccttttcata tattccacggcgctatcgccttgtgcattccatttttgaaagaacatcagctgttctgtgaccagcgggtaaaata ctgaaataccgatgataaacaaaaacagcttaaagcggatccacggtactccataataatggcgccacgccataat gaagccggcgatgatcataaacatgcagatcagaaccactaaattccggatgggcagttttaaaaagcccgaaagc gcgtctgctacagacgcgccttgatgattctcggcgctgatcaacaggccgataacaatcatgatgatgccggcaa gccctgtatgtaccttcatttctttccctcctcagttgtagaaatagtcgaacagagaggaaaaaggtttccggtt ttacttgaaaatgtcaaaccatcctttatgccggaaccagaagatcat |

### <1-2> Construction of recombinant B. licheniformis with deletion of epsAB, pgsBCAE, dgp, budC, and gdh

The DNA fragments prepared in <1-1> above were transferred to Bacillus licheniformis GSC4071 using electroporation (25 uF, 200 Ω, 2.5 kV/cm), and the target gene could be removed using the microbial recombination mechanism.

A DNA fragment containing the homologous region of the epsAB gene was transferred to wild-type Bacillus licheniformis GSC4071 to produce recombinant Bacillus licheniformis in which the epsAB gene was removed. The produced recombinant microorganism in which the epsAB gene was deleted was used as the recombinant strain of Example 1 (B. licheniformis 4071 ΔepsAB).

Meanwhile, after removing the epsAB gene from the wild-type Bacillus licheniformis, a DNA fragment containing the homologous region of the dgp gene was transferred to produce Bacillus licheniformis in which the dgp gene was removed in addition to the deletion of the epsAB gene. The produced recombinant microorganism in which the epsAB and dgp genes were deleted was used as the strain in Example 2 (B. licheniformis 4071 ΔepsAB Δdgp).

Then, after removing the epsAB gene from the wild-type Bacillus licheniformis, a DNA fragment containing the homologous region of the budC gene was transferred to produce Bacillus licheniformis in which the budC gene was removed in addition to the deletion of the epsAB gene. The produced recombinant microorganism in which the epsAB and budC genes were deleted was used as the strain in Example 3 (B. licheniformis 4071 ΔepsAB ΔbudC).

Then, after removing the epsAB gene from the wild-type Bacillus licheniformis, a DNA fragment containing the homologous region of the gdh gene was transferred to produce Bacillus licheniformis in which the gdh gene was removed in addition to the deletion of the epsAB gene. The produced recombinant microorganism in which the epsAB and gdh genes were deleted was used as the strain in Example 4 (B. licheniformis 4071 ΔepsAB Δgdh).

Then, after removing the epsAB and dgp genes from the wild-type Bacillus licheniformis, a DNA fragment containing the homologous region of the pgsBCAE gene was transferred to produce Bacillus licheniformis in which the pgsBCAE gene was removed in addition to the deletion of the epsAB and dgp genes. The produced recombinant microorganism in which the epsAB, dgp, and pgsBCAE genes were deleted was used as the strain in Example 5 (B. licheniformis 4071 ΔepsAB Δdgp ΔpgsBCAE).

Then, after removing the epsAB and gdh genes from the wild-type Bacillus licheniformis, a DNA fragment containing the homologous region of the pgsBCAE gene was transferred to produce Bacillus licheniformis in which the pgsBCAE gene was removed in addition to the deletion of the epsAB and gdh genes. The produced recombinant microorganism in which the epsAB, gdh, and pgsBCAE genes were deleted was used as the strain in Example 6 (B. licheniformis 4071 ΔepsAB Δgdh ΔpgsBCAE).

Then, after removing the epsAB, dgp, and pgsBCAE genes from the wild-type Bacillus licheniformis, a DNA fragment containing the homologous region of the budC gene was transferred to produce Bacillus licheniformes in which the budC gene removed in addition to the deletion of the epsAB, dgp, and pgsBCAE genes. The produced recombinant microorganism in which the epsAB, dgp, pgsBCAE, and budC genes were deleted was used as the strain in Example 7 (B. licheniformis 4071 ΔepsAB Δdgp ΔpgsBCAE ΔbudC).

Then, after removing the epsAB, dgp, and pgsBCAE genes from the wild-type Bacillus licheniformis, a DNA fragment containing the homologous region of the gdh gene was transferred to produce Bacillus licheniformes in which the gdh gene removed in addition to the deletion of the epsAB, dgp, and pgsBCAE genes. The produced recombinant microorganism in which the epsAB, dgp, pgsBCAE, and gdh genes were deleted was used as the strain in Example 8 (B. licheniformis 4071 ΔepsAB Δdgp ΔpgsBCAE Δgdh).

Then, the dgp gene was removed by transferring a DNA fragment containing the homologous region of the dgp gene from the wild-type Bacillus licheniformis, and then the DNA fragment containing the homologous region of the budC gene was transferred to produce Bacillus licheniformis in which the dgp gene and budC gene were removed. The produced recombinant microorganism in which dgp and budC genes were deleted was used as the strain in Example 9 (B. licheniformis 4071 Δdgp ΔbudC).

Then, the dgp gene was removed by transferring a DNA fragment containing the homologous region of the dgp gene from the wild-type Bacillus licheniformis, and then the DNA fragment containing the homologous region of the gdh gene was transferred to produce Bacillus licheniformis in which the dgp gene and gdh gene were removed. The produced recombinant microorganism in which the dgp and gdh genes were deleted was used as the strain in Example 10 (B. licheniformis 4071 Δdgp Δ gdh).

In the following Experimental Examples, the following culture and analysis experiments were performed using the recombinant microorganism produced in this way. In this case, the wild-type strain Bacillus licheniformis GSC4071 was used as Comparative Example 1.

**[Table 2]**

| Comparative Example 1 | wild-type strain Bacillus licheniformis GSC4071 |
|---|---|
| Example 1 | B. licheniformis 4071 ΔepsAB |
| Example 2 | B. licheniformis 4071 ΔepsAB Δdgp |
| Example 3 | B. licheniformis 4071 ΔepsAB ΔbudC |
| Example 4 | B. licheniformis 4071 ΔepsAB Δgdh |
| Example 5 | B. licheniformis 4071 ΔepsAB Δdgp ΔpgsBCAE |
| Example 6 | B. licheniformis 4071 ΔepsAB Δgdh ΔpgsBCAE |
| Example 7 | B. licheniformis 4071 ΔepsAB Δdgp ΔpgsBGAE ΔbudC |
| Example 8 | B. licheniformis 4071 ΔepsAB Δdgp ΔpgsBCAE Δgdh |
| Example 9 | B. licheniformis 4071 Δdgp ΔbudC |
| Example 10 | B. licheniformis 4071 Δdgp Δgdh |

### <Experimental Example 2> Evaluation of production ability of viscous polymer and polyol of microorganisms of Comparative Example 1 and Example 1 through low-temperature cultivation at 30°C

Using the wild-type Bacillus of Comparative Example 1 and the recombinant Bacillus strain of Example 1, the ability to simultaneously produce viscous polymer and polyols was evaluated.

Flask culture was performed on these microorganisms. Cultivation of the above microorganisms was performed as follows.

First, in order to determine the effect of the carbon source on the production of viscous polymer, culture was performed in media supplemented with glucose, glucose + glutamic acid, sucrose, and sucrose + glutamic acid, respectively.

The microorganisms were inoculated into a 250 ml flask containing 50 ml of complex medium containing 100 g/L glucose or sucrose at the same concentration and cultured at 30°C for 48 hours at 180 rpm. In addition, for polyglutamic acid production, 20 g/L glutamic acid was additionally added to the medium. Culture conditions were such that microaerobic conditions were maintained, and the culture was carried out at an initial pH of 7.0. Samples were collected from the wild-type and recombinant microorganisms after completion of cultivation. The collected samples were centrifuged at 13,000 rpm for 10 minutes to remove cells, and the supernatant was analyzed by liquid chromatography (HPLC) to analyze 2,3-Butanediol and glycerol. The viscous polymer concentration of the supernatant was first mixed with ethanol at a ratio of 1:2, then subjected to ethanol precipitation for 24 hours, and the polymer was recovered by centrifugation. Low molecules were removed from the recovered polymer through dialysis (membrane dialysis, 10 kDa), and finally, the dry weight was measured through freeze drying. In order to identify polyglutamic acid, levan, and EPS among the dried viscous polymers, they were identified through NMR analysis.

As a result, when the wild-type microorganism of Comparative Example 1 was cultured in glucose, the total polyol was 38.57 g/L, glycerol was 8.09 g/L, (2R,3R)-butanediol was 16.48 g/L, (2R,3S)-butane Diol was 14 g/L. It was confirmed that the total viscous polymer was 9.71 g/L, and all of them were EPS. Therefore, when the wild-type microorganism of Comparative Example 1 was cultured in glucose at a low temperature of 30°C, it was confirmed that all three polyols were produced, and only EPS was selectively produced as the viscous polymer.

When the wild-type microorganism of Comparative Example 1 was cultured in glucose + glutamic acid medium, the total polyol was 38.12 g/L, glycerol was 8.24 g/L, (2R,3R)-butanediol was 13.80 g/L, (2R,3S)-butanediol was 16.08 g/L. The total viscous polymer of 10.71 g/L was produced, of which PGA of 7.22 g/L (67.4%) was produced and EPS of 3.49 g/L (32.6%) was produced. Therefore, it was confirmed that PGA could be produced with high selectivity only when glutamic acid was added to the medium when culturing the wild-type microorganism of Comparative Example 1 at a low temperature of 30°C.

When the wild-type microorganism of Comparative Example 1 was cultured in sucrose, the total polyol was 35.61 g/L, of which glycerol was 7.97 g/L, (2R,3R)-butanediol was 13.78 g/L, and (2R,3S)-butanediol was 13.85 g/L. The total viscous polymer was 13.65 g/L, of which levan was 11.18 g/L (81.9%) and EPS was 2.47 g/L (18.1%). Therefore, it was confirmed that when the wild-type microorganism of Comparative Example 1 was cultured in sucrose, levan was predominantly produced.

Finally, when the wild-type microorganism of Comparative Example 1 was cultured in sucrose + glutamic acid medium, the total polyol was 37.51 g/L, of which glycerol was 9.76 g/L, (2R,3R)-butanediol was 11.23 g/L, (2R,3S)-butanediol was 16.52 g/L. The total viscous polymer was 16.18 g/L, of which PGA was 1.65 g/L (10.2%), levan was 10.40 g/L (64.3%), and EPS was 4.13 g/L (25.5%). It was confirmed that all three polyols and three viscous polymers were produced under these culture conditions.

Next, a culture experiment was conducted using the recombinant microorganism of Example 1.

When the recombinant microorganism of Example 1 was cultured in glucose medium, the total polyol was 39.72 g/L, of which glycerol was 6.77 g/L, (2R,3R)-butanediol was 17.87 g/L, (2R, 3S)- butanediol was 15.09 g/L. It was confirmed that the total viscous polymer was at the level of 0.88 g/L, and all of them were EPS. This shows that EPS production may be largely inhibited through deletion of the epsAB gene.

As a result of culturing the recombinant microorganism of Example 1 in glucose + glutamic acid medium, the total polyol was 41.67 g/L, of which glycerol was 8.31 g/L, (2R, 3R)-butanediol was 16.22 g/L, (2R, 3S)-butanediol was 17.14 g/L. The total viscous polymer was 1.82 g/L, of which PGA was 1.30 g/L (71.4%) and EPS was 0.52 g/L (28.6%). By removing the epsAB gene, the PGA production ratio slightly increased, but the overall amount of viscous polymer production was confirmed to be reduced. This was determined to be the effect of low-temperature cultivation at 30°C, and additional experiments were conducted in Experimental Example 2.

Finally, as a result of culturing the recombinant microorganism of Example 1 in sucrose medium, the total polyol was 41.23 g/L, of which glycerol was 8.73 g/L, (2R,3R)-butanediol was 16.66 g/L, (2R,3S)-butanediol was 15.83 g/L. The total viscous polymer was 19.29 g/L, of which levan was 18.72 g/L (97%) and EPS was 0.58 g/L (3%). Therefore, it was confirmed that the selectivity and production amount of levan were significantly increased through deletion of the epsAB gene. Through this, it was confirmed that under low-temperature culture conditions at 30°C, there was no significant change in polyol production due to medium components and deletion of the epsAB gene, but the viscous polymer was significantly affected.

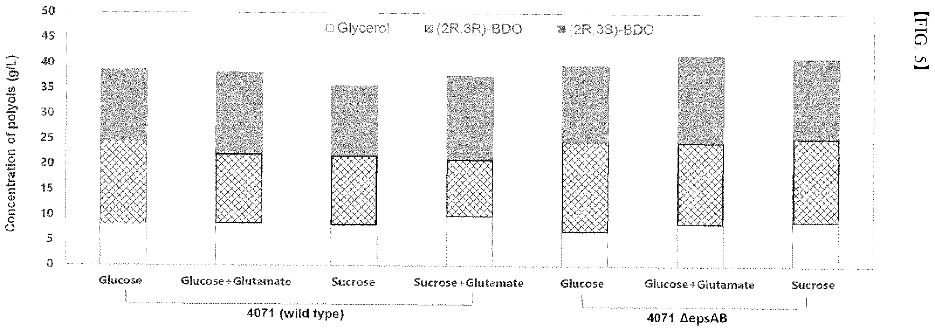

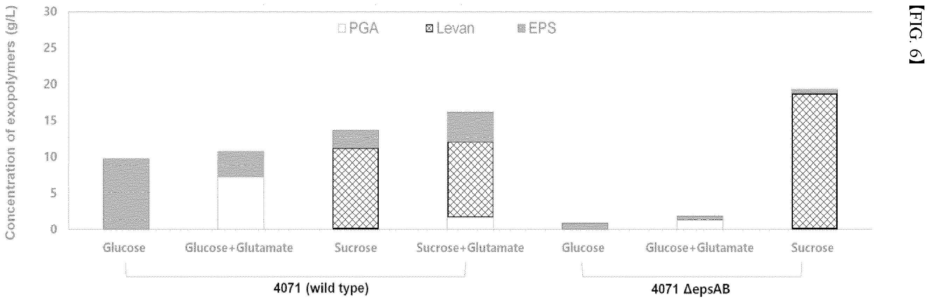

As a result, the wild-type microorganism of Comparative Example 1 was able to selectively produce polyol and EPS in glucose medium. In the case of PGA under low-temperature culture conditions at 30°C, an improvement in selectivity was observed due to deletion of the epsAB gene, and it was found that the production amount was not high. However, as a result of culturing the recombinant microorganism of Example 1 in which the epsAB gene was deleted at 30°C in a sucrose-containing medium, it was confirmed that levan could be produced with high selectivity. (Tables 3 and 4, FIGS. 5 and 6).

**[Table 3]**

| **Carbon source** | **Glucose** | **Glucose + Glutamic acid** | **Sucrose** | **Sucrose + Glutamic acid** |
|---|---|---|---|---|
| **Total polyol (g/L)** | **38.57** | **38.12** | **35.61** | **37.51** |
| Glycerol | 8.09 | 8.24 | 7.97 | 9.76 |
| (2R,3R)-butanediol | 16.48 | 13.80 | 13.78 | 11.23 |
| (2R,3S)-butanediol | 14.00 | 16.08 | 13.85 | 16.52 |
| **Total viscous polymer (g/L)** | **9.71** | **10.71** | **13.65** | **16.18** |
| PGA | 0.00 | 7.22 | 0.00 | 1.65 |
| Levan | 0.00 | 0.00 | 11.18 | 10.40 |
| EPS | 9.71 | 3.49 | 2.47 | 4.13 |

Analysis of cultures of wild-type microorganisms of Comparative Example 1 according to carbon source

**[Table 4]**

| **Carbon source** | **Glucose** | **Glucose + Glutamic acid** | **Sucrose** |
|---|---|---|---|
| **Total polyol (g/L)** | **39.72** | **41.67** | **41.23** |
| Glycerol | 6.77 | 8.31 | 8.73 |
| (2R,3R)-butanediol | 17.87 | 16.22 | 16.66 |
| (2R,3S)-butanediol | 15.09 | 17.14 | 15.83 |
| **Total viscous polymer (g/L)** | **0.88** | **1.82** | **19.29** |
| PGA | 0.00 | 1.30 | 0.00 |
| Levan | 0.00 | 0.00 | 18.72 |
| EPS | 0.88 | 0.52 | 0.58 |

Analysis of cultures of recombinant microorganisms of Example 1 according to carbon source

### <Experimental Example 3> Evaluation of production ability of viscous polymer and polyol production of microorganisms of Comparative Example 1 and Example 1 through high temperature cultivation at 45°C

The wild-type Bacillus strain of Comparative Example 1 and the recombinant Bacillus strain of Example 1 were cultured under high temperature conditions at 45°C to evaluate changes in the production of polyol and viscous polymer. All basic conditions were the same as in Example 1, and only the culture temperature was changed to 45°C.

As a result, when the wild-type microorganism of Comparative Example 1 was cultured at high temperature in glucose medium, the total polyol was 45.15 g/L, of which glycerol was 8.85 g/L, (2R, 3R)-butanediol was 13.6 g/L, (2R, 3S)-butanediol was 22.7 g/L. As a result, it was confirmed that the total amount of polyol produced by high-temperature culture increased compared to low-temperature culture. The total viscous polymer was 5.06 g/L, of which PGA was 3.69 g/L and EPS was 1.37 g/L.

When the wild-type microorganism of Comparative Example 1 was cultured at high temperature in a glucose + glutamic acid medium, the total polyol was 44.81 g/L, of which glycerol was 8.85 g/L, (2R, 3R)-butanediol was 10.6 g/L, (2R, 3S )-butanediol was 25.2 g/L. The total viscous polymer was 5.47 g/L, of which PGA was 3.98 g/L and EPS was 1.49 g/L. In low-temperature cultivation of the wild-type microorganism of Comparative Example 1, it was confirmed that no PGA was produced at all when using a medium without glutamic acid, but in high-temperature cultivation, PGA production was possible even without the addition of glutamic acid.

Next, a culture experiment was performed using the recombinant microorganism of Example 1.

As a result of producing the recombinant microorganism of Example 1 in glucose medium, the total polyol production was 50.1 g/L, of which glycerol was 11.5 g/L, (2R, 3R)-butanediol was 12.8 g/L, (2R, 3S)-butanediol was 25.2 g/L. The total viscous polymer was 2.65 g/L, of which PGA was 1.59 g/L and EPS was 1.05 g/L. It was confirmed that epsAB was deleted upon high-temperature culture, and PGA was produced even in a medium without added glutamic acid.

As a result of culturing the recombinant microorganism of Example 1 in glucose + glutamic acid medium, total polyol was 48.12 g/L, of which glycerol was 10.02 g/L, (2R,3R)-butanediol was 11.5 g/L, (2R,3S)butanediol was 26.6 g/L. The total viscous polymer was 5.45 g/L, of which PGA was 5.10 g/L and EPS was 0.35 g/L. It was confirmed that when the recombinant microorganism of Example 1 was cultured in glucose + glutamic acid medium, the selectivity of PGA was 93%, and the production of PGA was increased by 73% compared to that of the wild-type microorganism of Comparative Example 1. In addition, it was confirmed that when the recombinant microorganism of Example 1 was cultured at high temperature, not only the selectivity but also the production ratio of PGA increased significantly.

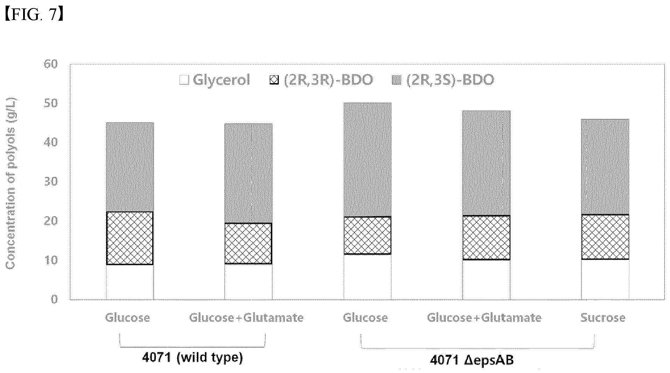

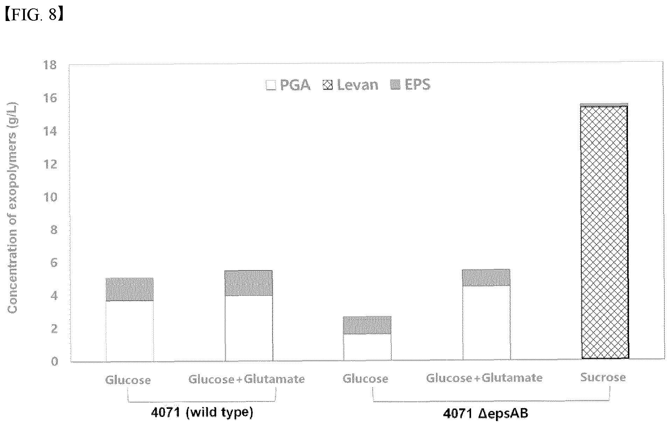

Finally, as a result of culturing the recombinant microorganism of Example 1 in sucrose, total polyol 46 g/L, of which glycerol was 10.2 g/L, (2R,3R)-butanediol was 11.5 g/L, (2R,3S)-butanediol was 24.3 g/L. The total viscous polymer was 15.47 g/L, of which levan was 15.31 g/L and EPS was 0.154 g/L. In the case of levan, it was confirmed that high selectivity and high production amount were observed under both low and high temperature conditions. (Table 5, FIGS. 7 and 8).

**[Table 5]**

| | **Comparative Example 1** | | **Example 1** | | |
|---|---|---|---|---|---|
| | **Glucose** | **Glucose + Glutamic acid** | **Glucose** | **Glucose + glutamic acid** | **Sucrose** |
| **Total polyol (g/L)** | **45.15** | **44.81** | **50.1** | **48.12** | **46.00** |
| Acetoin (g/L) | 1.68 | 3.45 | 1.29 | 2.48 | 2.30 |
| Glycerol | 8.85 | 9.01 | 11.5 | 10.02 | 10.20 |
| (2R,3R)- butanediol | 13.6 | 10.6 | 12.8 | 11.5 | 11.50 |
| (2R,3S)-butanediol | 22.7 | 25.2 | 25.8 | 26.6 | 24.30 |
| **Total viscous polymer (g/L)** | **5.06** | **5.47** | **2.65** | **5.45** | **15.47** |
| PGA | 3.69 | 3.98 | 1.59 | 5.10 | 0 |
| Levan | 0 | 0 | 0 | 0 | 15.31 |
| EPS | 1.37 | 1.49 | 1.05 | 0.35 | 0.154 |

### <Experimental Example 4> Evaluation of recombinant microorganism culture with improved selectivity of levan and polyol

In Experimental Examples 2 and 3, the improvement in production and selectivity of the viscous polymer was evaluated, and it was confirmed that levan had the highest production and selectivity among the viscous polymers. Accordingly, it was attempted to confirm whether selective simultaneous production of levan and a specific polyol was possible by performing additional genetic recombination to the wild-type microorganism of Comparative Example 1. During cultivation, sucrose was used as a carbon source, and other experimental conditions were performed under the same conditions as in Experimental Example 1.

As a result, the recombinant microorganism of Example 2 was produced to inhibit glycerol production, and as a result of culture, it was confirmed that among polyols, glycerol was not performed at all. The recombinant microorganism of Example 3 was prepared to inhibit (2R, 3S)-butanediol production, and as a result of cultivation, it was confirmed that it hardly produces (2R, 3S)-butanediol among polyols. The recombinant microorganism of Example 4 was prepared to inhibit (2R, 3R)-butanediol production, and as a result of cultivation, it was confirmed that it hardly produces (2R, 3R)-butanediol among polyols.

The recombinant microorganism of Example 3 was confirmed to produce 23.85 g/L of levan, but it was confirmed that the levan production of the recombinant microorganism of Examples 2 and 4 was significantly inhibited by deleting a specific polyol production gene. As a result, it was confirmed that the deletion of genes involved in the production of specific polyols is linked to the inhibition of viscous polymer production through a regulatory mechanism between the components that constitute the biofilm.

This problem was solved by constructing the recombinant microorganism of Example 5 in which the pgsBCAE gene was additionally deleted from the recombinant microorganism of Example 2. The pgsBCAE gene is a PGA synthetase, and PGA is one of the components that form biofilm. It was experimentally confirmed that additionally deleting the pgsBCAE gene could restore the ability to produce the viscous polymer.

As a result of culturing the recombinant microorganism of Example 5, it was confirmed that the total polyol of 29.87 g/L was produced, of which (2R, 3R)-butanediol of 16.15 g/L was produced, (2R, 3S)-butanediol of 13.71 g/L was produced, and no glycerol was produced. It was confirmed that the total viscous polymer of 22.18 g/L was produced, of which levan of 21.91 g/L was produced, and EPS of 0.27 g/L was produced. It was confirmed that selective simultaneous production of polyol and levan from which glycerol was removed was possible through the recombinant microorganism of Example 5.

The recombinant microorganism of Example 6 was produced by additionally deleting pgsBCAE from the recombinant microorganism of Example 4. As a result, the total polyol of 35.4 g/L was produced, of which glycerol of 7.29 g/L was produced, and (2R,3S)-butanediol of 28.1 g/L was produced. The total viscous polymer of 25.59 g/L was produced, of which levan of 24.82 g/L was produced, and EPS of 0.77 g/L was produced. It was confirmed that polyols and levan, excluding (2R,3R)-butanediol, could be selectively produced simultaneously.

The recombinant microorganism of Example 7 was produced by additionally deleting the budC gene from the recombinant microorganism of Example 5, in which the glycerol production ability was inhibited and the viscous polymer production ability was restored. As a result of culturing the recombinant microorganism of Example 7, the total polyol was 25.47 g/L, of which glycerol was 0, (2R,3R)-butanediol was 25.34 g/L (99.5%), (2R,3S)-butanediol was 0.14 g /L (0.5%). As a result of culturing the recombinant microorganism of Example 7, the total viscous polymer was 23.94 g/L, of which levan was 23.15 g/L (96.7%) and EPS was 0.79 g/L (3.3%). Finally, by culturing the recombinant microorganism of Example 7, it was confirmed that high-selectivity (2R,3R)-butanediol and high-selectivity levan could be produced simultaneously.

The recombinant microorganism of Example 8 was produced by additionally deleting the gdh gene from the recombinant microorganism of Example 5, in which the glycerol production ability was inhibited and the viscous polymer production ability was restored. As a result of culturing the recombinant microorganism of Example 8, the total polyol was 25.4 g/L, of which glycerol was 0, (2R,3R)-butanediol was 0, and (2R,3S)-butanediol was 25.4 g/L (100%). As a result of culturing the recombinant microorganism of Example 8, the total viscous polymer was 23.59 g/L, of which levan was 22.41 g/L (95%), and EPS was 1.18 g/L (5%). Finally, it was confirmed that high-selectivity (2R,3S)-butanediol and high-selectivity levan could be simultaneously produced by culturing the recombinant microorganism of Example 8 (Table 6, FIG.s 9 and 10).

**[Table 6]**

| | **Compa rative Examp le 1** | **Examp le 1** | **Examp le 2** | **Examp le 3** | **Examp le 4** | **Examp le 5** | **Examp le 6** | **Examp le 7** | **Examp le 8** |
|---|---|---|---|---|---|---|---|---|---|
| **Total polyol (g/L)** | **35.61** | **41.23** | **32.85** | **28.72** | **30.95** | **29.87** | **35.40** | **25.47** | **25.40** |
| Glycerol (g/L) | 7.97 | 8.73 | 0.00 | 5.13 | 6.59 | 0.00 | 7.29 | 0.00 | 0.00 |
| (2R,3R)-butanediol (g/L) | 13.78 | 16.66 | 18.35 | 23.36 | 0.00 | 16.15 | 0.00 | 25.34 | 0.00 |
| (2R,3S)-butanediol (g/L) | 13.85 | 15.83 | 14.50 | 0.23 | 24.36 | 13.71 | 28.10 | 0.14 | 25.40 |
| **Total viscous polymer(g /L)** | **13.65** | **19.29** | **0.89** | **24.59** | **0.76** | **22.18** | **25.59** | **23.94** | **23.59** |
| PGA | 0.00 | 0.00 | - | - | - | - | - | - | - |
| Levan | 11.18 | 18.72 | 0.36 | 23.85 | 0.51 | 21.91 | 24.82 | 23.15 | 22.41 |
| EPS | 2.47 | 0.58 | 0.54 | 0.74 | 0.26 | 0.27 | 0.77 | 0.79 | 1.18 |

### <Experimental Example 5> Evaluation of recombinant microorganism culture with improved PGA and polyol selectivity

In Experimental Example 3, the ability to produce viscous polymers and polyols was evaluated through high-temperature cultivation of microorganisms at 45°C. When cultured in a glucose medium, it was configured in Example 1 that PGA was possible to be produced without the addition of glutamic acid, and high selectivity and production of PGA among viscous polymers could be obtained when glutamic acid was added. An experiment was conducted to confirm the possibility of selective simultaneous production of PGA and polyol based on the microorganism of Example 1. This experiment was conducted through high-temperature cultivation at 45°C in glucose medium supplemented with glutamic acid. Comparative evaluation was conducted on Example 2, Example 3, and Example 4.

As a result of culturing the recombinant microorganism of Example 2, it was confirmed that the total polyol of 31.8 g/L was produced, of which (2R,3R)-butanediol of 10.6 g/L of was produced, and (2R,3S)-butanediol of 21.2 g/L was produced, and no glycerol was produced. It was confirmed that the total viscous polymer was 0.35 g/L, and EPS was produced in all of them. When dgp, a glycerol production gene, was deleted, it was confirmed that the production of viscous polymer, including PGA, was significantly inhibited.

As a result of culturing the recombinant microorganism of Example 3, the total polyol of 48.3 g/L was produced, of which (2R,3R)-butanediol of 35.4 g/L was produced, (2R,3S)-butanediol of 1.1 g/L was produced, and glycerol was 11.8 g/L. It was confirmed that (2R,3R)-butanediol could be produced with high selectivity. It was confirmed that the total viscous polymer of 6.35 g/L was produced, of which PGA of 6.15 g/L was produced and EPS of 0.20 g/L was produced. Overall, when budC, the (2R,3S)-butanediol production gene, was deleted, it was confirmed that (2R,3R)-butanediol, glycerol, and PGA could be selectively produced simultaneously.

As a result of culturing the recombinant microorganism of Example 4, the total polyol of 37.2 g/L was produced, of which 35.0 g/L of (2R,3S)-butanediol, and 2.2 g/L of glycerol were produced. (2R,3R)-butanediol was not produced at all. It was confirmed that the total viscous polymer was 0.45 g/L, and EPS was produced in all of them. In Example 4, it was confirmed that when gdh, the (2R,3R)-butanediol producing gene, was deleted, the production of viscous polymers, including PGA, was significantly inhibited (Table 7, FIG.s 11 and 12).

**[Table 7]**

| | **Example 2** | **Example 3** | **Example 4** |
|---|---|---|---|
| **Total polyol (g/L)** | **31.8** | **48.3** | **37.2** |
| Glycerol (g/L) | 0 | 11.8 | 2.2 |
| (2R,3R)- butanediol (g/L) | 10.6 | 35.4 | 0 |
| (2R,3S)- butanediol (g/L) | 21.2 | 1.1 | 35 |
| **Total viscous polymer (g/L)** | **0.35** | **6.35** | **0.45** |
| PGA | 0 | 6.15 | 0 |
| Levan | 0 | 0 | 0 |
| EPS | 0.35 | 0.20 | 0.45 |

### <Experimental Example 6> Evaluation of recombinant microorganism culture with improved EPS and polyol selectivity

In Experimental Example 2, it was confirmed that only EPS among the viscous polymers could be produced when the microorganism of Comparative Example 1 was cultured at a low temperature of 30°C in glucose medium. To confirm the possibility of selective simultaneous production of EPS and (2R,3R)-butanediol or (2R,3S)-butanediol, a culture experiment was conducted using the microorganisms of Examples 9 and 10. This experiment was conducted through low-temperature cultivation at 30°C in glucose medium.

As a result of culturing the recombinant microorganism of Example 9, the total polyol of 28.1 g/L was produced, of which 27.8 g/L of (2R, 3R)-butanediol, and 0.3 g/L of (2R,3S)-butanediol were produced. It was confirmed that no glycerol was produced at all, and (2R,3R)-butanediol could be produced with high selectivity. It was confirmed that the total viscous polymer was 7.22 g/L, and all of them were EPS. Through this, it was confirmed that (2R,3R)-butanediol and EPS could be selectively produced simultaneously.

As a result of culturing the recombinant microorganism of Example 10, it was confirmed that the total polyol was 26.1 g/L, and all of them were (2R,3S)-butanediol. It was confirmed that (2R,3R)-butanediol and glycerol were not produced at all, and (2R,3S)-butanediol could be produced with high selectivity. It was confirmed that the total viscous polymer was 6.56 g/L, and all of them were EPS. Through this, it was confirmed that (2R,3S)-butanediol and EPS could be selectively produced simultaneously (Table 8, FIG.s 13 and 14).

**[Table 8]**

| | **Example 9** | **Example 10** |
|---|---|---|
| **Total polyol (g/L)** | **28.1** | **26.1** |
| Glycerol (g/L) | 0 | 0 |
| (2R,3R)- butanediol (g/L) | 27.8 | 0 |
| (2R,3S)- butanediol (g/L) | 0.3 | 26.1 |
| **Total viscous polymer (g/L)** | **7.22** | **6.56** |
| PGA | 0 | 0 |
| Levan | 0 | 0 |
| EPS | 7.22 | 6.56 |

### <Experimental Example 7> Fed-batch fermentation culture for selective simultaneous production of levan and (2R, 3S)-butanediol

To verify the ability to simultaneously produce levan and (2R, 3S)-butanediol with high selectivity, the recombinant microorganism of Example 8 was subjected to fed-batch fermentation in sucrose medium conditions to evaluate the fermentation performance.

The microbial fermentation experiment was performed as follows.

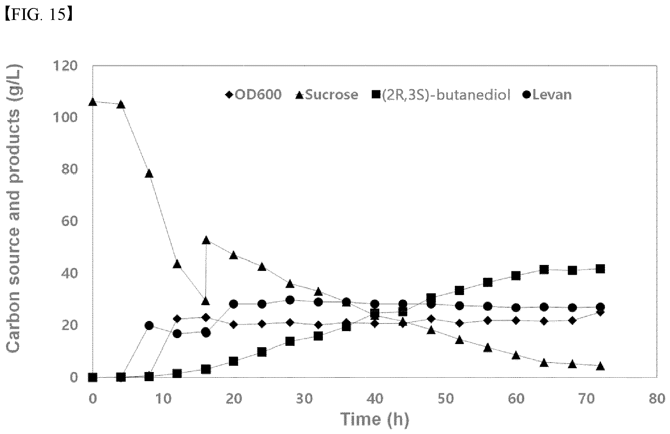

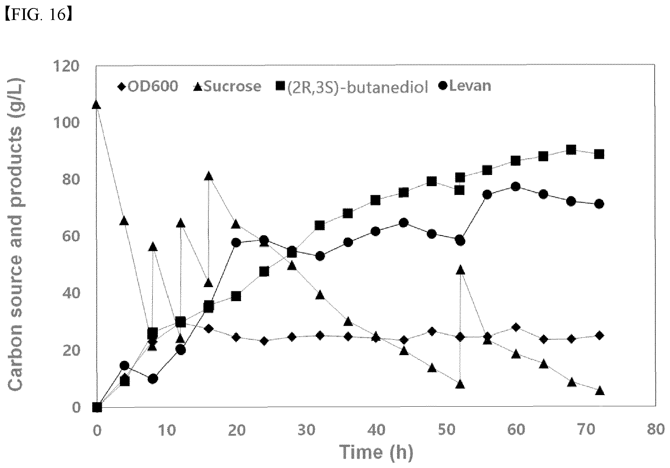

First, the recombinant microorganism of Example 8 was inoculated into a 1L flask containing 300 ml of complex medium and cultured at 30°C or 45°C for 16 hours at 180 rpm. The cultured microorganisms were inoculated into a 7.5L fermentor so that the final culture volume was 3L. The initial conditions were pH 7.0, temperature 30°C or 45°C, 550 rpm, and oxygen 1vvm. The initial sucrose concentration was set to 100 g/L. During the culture process, when the amount of acetoin became more than 10 g/L, a second stage fermentation was performed by lowering the stirring speed to 350 rpm. When the sugar concentration in the culture medium fell below 20 g/L, the fermentation was conducted by additionally feeding 150 g of sucrose powder. Samples were collected at 4-hour intervals during fermentation, and cell growth was measured using a spectrophotometer as an OD600 value. The collected samples were centrifuged at 13,000 rpm for 10 minutes, and the (2R,3S)-butanediol concentration of the supernatant was analyzed by liquid chromatography (HPLC). Levan was recovered through ethanol precipitation and quantified through NMR analysis. As a result, when low-temperature culture fed-batch fermentation was performed at 30°C, (2R, 3S)-butanediol production was 41.8 g/L and levan production was 27 g/L. On the other hand, when high-temperature culture fed-batch fermentation was performed at 45°C, (2R,3S)-butanediol production was 88.6 g/L and levan was 71 g/L. Levan and (2R,3S)-butanediol among viscous polymers and polyols were produced with a selectivity of over 99% under both low and high temperature culture conditions. Compared to low-temperature culture, the production of levan and (2R,3S)-butanediol was about twice as high in high-temperature culture. This was found to be because the recombinant microorganism of Example 8 grew rapidly at high temperature and had a much faster metabolic rate. In addition, it was confirmed that industrial-level high-concentration production was achieved in the selective simultaneous production of viscous polymer and polyol. (Table 9, FIGS. 15 and 16).

**[Table 9]**

| | (2R,3S)- butanediol (g/L) | Polyol selectivity (%) | Levan (g/L) | Viscous polymer selectivity (%) |
|---|---|---|---|---|
| 30°C fermentation | 41.8 | >99% | 27 g/L | >99% |
| 45°C fermentation | 88.6 g/L | >99% | 71 g/L | >99% |

### [Industrial Applicability]

The present invention relates to a recombinant microorganism with a controlled ability to produce polyol or exopolymer. In addition, the present invention relates to a method for producing polyol or exopolymer using the recombinant microorganism of the present invention.

### [SEQUENCE LISTING FREETEXT]

SEQ ID NO: 1: Base sequence of epsAB
SEQ ID NO: 2: Base sequence of homology region 1 of epsAB
SEQ ID NO: 3: Base sequence of a primer for amplifying homologous region 1 of epsAB by PCR
SEQ ID NO: 4: Base sequence of a primer for amplifying homologous region 1 of epsAB by PCR
SEQ ID NO: 5: Base sequence of homology region 2 of epsAB
SEQ ID NO: 6: Base sequence of a primer for amplifying homologous region 2 of epsAB by PCR
SEQ ID NO: 7: Base sequence of a primer for amplifying homologous region 2 of epsAB by PCR
SEQ ID NO: 8: Base sequence of a DNA fragment containing homology regions 1 and 2 of epsAB
SEQ ID NO: 9: Base sequence of pgsBCAE
SEQ ID NO: 10: Base sequence of homology region 1 of pgsBCAE
SEQ ID NO: 11: Base sequence of a primer for amplifying homologous region 1 of pgsBCAE by PCR
SEQ ID NO: 12: Base sequence of a primer for amplifying homologous region 1 of pgsBCAE by PCR
SEQ ID NO: 13: Base sequence of homology region 2 of pgsBCAE
SEQ ID NO: 14: Base sequence of a primer for amplifying homologous region 2 of pgsBCAE by PCR
SEQ ID NO: 15: Base sequence of a primer for amplifying homologous region 2 of pgsBCAE by PCR
SEQ ID NO: 16: Base sequence of a DNA fragment containing homology regions 1 and 2 of pgsBCAE
SEQ ID NO: 17: Base sequence of dgp
SEQ ID NO: 18: Base sequence of homology region 1 of dgp
SEQ ID NO: 19: Base sequence of a primer to amplify homologous region 1 of dgp by PCR
SEQ ID NO: 20: Base sequence of a primer to amplify homologous region 1 of dgp by PCR
SEQ ID NO: 21: Base sequence of homology region 2 of dgp
SEQ ID NO: 22: Base sequence of a primer to amplify homologous region 2 of dgp by PCR
SEQ ID NO: 23: Base sequence of a primer to amplify homologous region 2 of dgp by PCR
SEQ ID NO: 24: Base sequence of a DNA fragment containing homology regions 1 and 2 of dgp
SEQ ID NO: 25: Base sequence of budC
SEQ ID NO: 26: Base sequence of homology region 1 of budC
SEQ ID NO: 27: Base sequence of a primer to amplify homologous region 1 of budC by PCR
SEQ ID NO: 28: Base sequence of a primer to amplify homologous region 1 of budC by PCR
SEQ ID NO: 29: Base sequence of homology region 2 of budC
SEQ ID NO: 30: Base sequence of a primer to amplify homologous region 2 of budC by PCR
SEQ ID NO: 31: Base sequence of a primer to amplify homologous region 2 of budC by PCR
SEQ ID NO: 32: Base sequence of a DNA fragment containing homology regions 1 and 2 of budC
SEQ ID NO: 33: Base sequence of gdh
SEQ ID NO: 34: Base sequence of homology region 1 of gdh
SEQ ID NO: 35: Base sequence of a primer to amplify homologous region 1 of gdh by PCR
SEQ ID NO: 36: Base sequence of a primer to amplify homologous region 1 of gdh by PCR
SEQ ID NO: 37: Base sequence of homology region 2 of gdh
SEQ ID NO: 38: Base sequence of a primer to amplify homologous region 2 of gdh by PCR
SEQ ID NO: 39: Base sequence of a primer to amplify homologous region 2 of gdh by PCR
SEQ ID NO: 40: Base sequence of a DNA fragment containing homology regions 1 and 2 of gdh
Name of depository institution: Korea Research Institute of Bioscience and Biotechnology
Deposit accession number: KCTC14485BP
Deposit date: 20210305

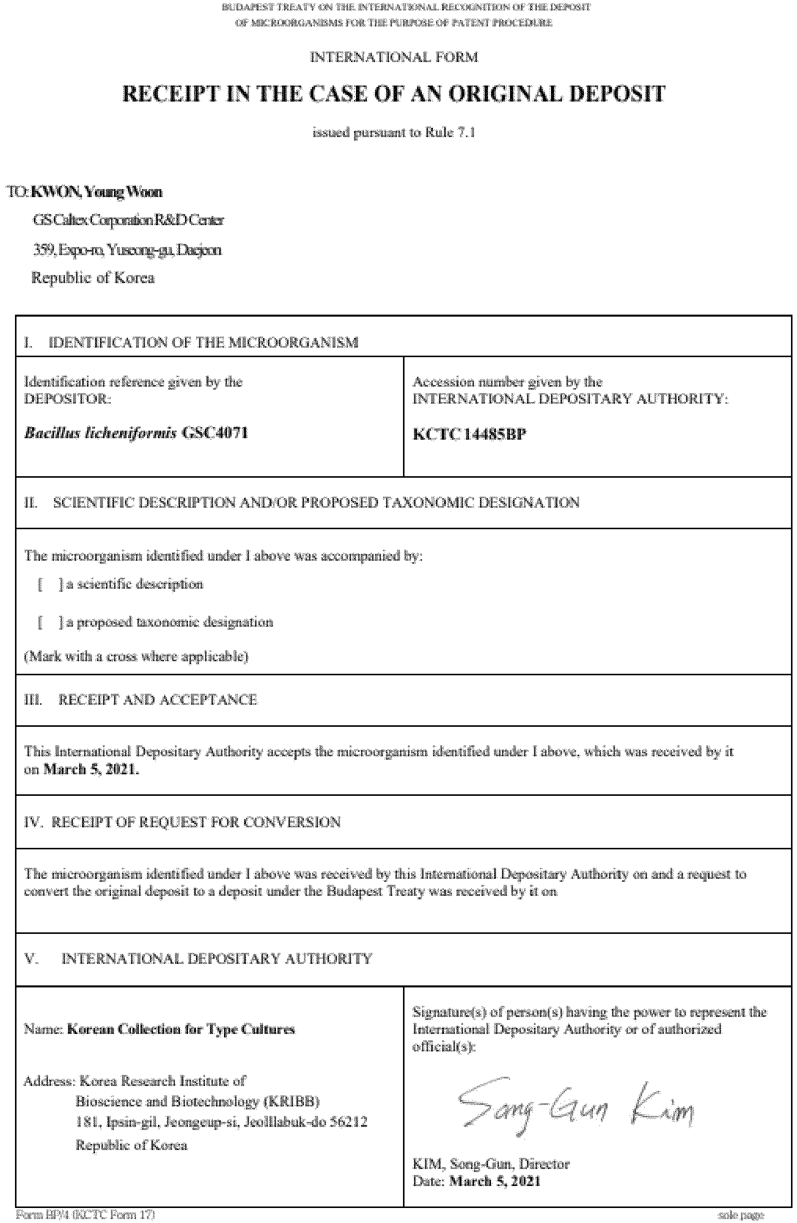
BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: KWON, Young Woon
GS Caltex Corporation R&D Center
359, Expo-ro, Yuseong-gu, Daejeon
Republic of Korea

I. IDENTIFICATION OF THE MICROORGANISM
Identification reference given by the DEPOSITOR: | Accession number given by the INTERNATIONAL DEPOSITARY AUTHORITY:
Bacillus licheniformis GSC4071 | KCTC 14485BP

II. SCIENTIFIC DESCRIPTION AND/OR PROPOSED TAXONOMIC DESIGNATION

The microorganism identified under I above was accompanied by:
[ ] a scientific description
[ ] a proposed taxonomic designation
(Mark with a cross where applicable)

III. RECEIPT AND ACCEPTANCE

This International Depositary Authority accepts the microorganism identified under I above, which was received by it
on March 5, 2021.

IV. RECEIPT OF REQUEST FOR CONVERSION

The microorganism identified under I above was received by this International Depositary Authority on and a request to
convert the original deposit to a deposit under the Budapest Treaty was received by it on

V. INTERNATIONAL DEPOSITARY AUTHORITY

Name: Korean Collection for Type Cultures | Signature(s) of person(s) having the power to represent the International Depositary Authority or of authorized official(s):

Address: Korea Research Institute of
Bioscience and Biotechnology (KRIBB)
181, Ipsin-gil, Jeongeup-si, Jeollabuk-do 56212
Republic of Korea |
KIM, Song-Gun, Director
Date: March 5, 2021

Form BP/4 (KCTC Form 17) sole page

## Claims

1. A recombinant microorganism with controlled ability to produce polyol or exopolymer in a microorganism with a biosynthetic pathway for polyol or exopolymer, **characterized in that** one or more selected from the group consisting of a pathway that converts glucose-6-phosphate to exopolysaccharide, a pathway that converts glycerophosphate to glycerol, a pathway that converts acetoin to (2R,3S)-butanediol, a pathway that converts acetoin to (2R,3R)-butanediol, and a pathway that converts glutamic acid to polyglutamic acid are inhibited.

2. The recombinant microorganism of claim of claim 1, wherein the recombinant microorganism is a recombinant Bacillus.

3. The recombinant microorganism of claim 1, wherein the polyol is one or more selected from the group consisting of glycerol, (2R, 3R)-butanediol, and (2R,3S)-butanediol.

4. The recombinant microorganism of claim 1, wherein the exopolymer is one or more selected from the group consisting of polyglutamic acid, levan, and exopolysaccharide.

5. The recombinant microorganism of claim 4, wherein the exopolysaccharide contains one or more monomers selected from the group consisting of glucose, galactose, phosphate, glycerol, and acetic acid.

6. The recombinant microorganism of claim 1, wherein compared to a wild-type microorganism, an ability to produce one or more polyols selected from the group consisting of glycerol, (2R, 3R)-butanediol, and (2R,3S)-butanediol are inhibited or the ability to produce the one or more polyols is not present.

7. The recombinant microorganism of claim 1, wherein the pathway that converts glucose-6-phosphate to exopolysaccharide is inhibited,
one or more selected from the group consisting of the pathway that converts glycerophosphate to glycerol, the pathway that converts acetoin to (2R,3S)-butanediol, the pathway that converts acetoin to (2R,3R)-butanediol, and the pathway that converts glutamic acid to polyglutamic acid is inhibited.

8. The recombinant microorganism of claim 1, wherein the recombinant microorganism has the ability to simultaneously produce the polyol and the exopolymer.

9. A method for producing polyol comprising the steps of:
preparing the recombinant microorganism of claim 1; and
culturing the recombinant microorganism in a medium containing a carbon source.

10. A method for producing exopolymer comprising the steps of:
preparing the recombinant microorganism of claim 1; and
culturing the recombinant microorganism in a medium containing a carbon source.

11. The method of claim 9 or 10, wherein the carbon source includes one or more selected from the group consisting of sucrose, glucose, and glutamic acid.

12. The method of claim 9 or 10, wherein the culture is carried out at 25 to 55 ° C.
